# EUROPEAN PATENT SPECIFICATION

(11) **EP 4 013 485 B1**
(45) Date of publication and mention of the grant of the patent: **08.04.2026**
(21) Application number: 20854966.7
(22) Date of filing: 18.08.2020
(51) Int. Cl.: A61N 1/04, A61N 1/18, A61N 1/36

(54) **METHODS FOR TRANSDERMAL NEUROSTIMULATION TREATMENT**
VERFAHREN ZUR TRANSDERMALEN NEUROSTIMULATIONSBEHANDLUNG
PROCÉDÉS DE TRAITEMENT PAR NEUROSTIMULATION TRANSDERMIQUE

(30) Priority: 18.08.2019 US 201962888497 P
(43) Date of publication of application: 22.06.2022
(73) Proprietor: Neurolief Ltd., 42504412 Netanya (IL)
(72) Inventor: DAR, Amit, 4069200 Kfar-Hess (IL); BELSON, Ron, 6291412 Tel Aviv (IL); COHEN, Amir, 4358818 Ra'anana (IL)
(74) Representative: Harrison IP Limited
(86) International application number: PCT/IB2020/057781
(87) International publication number: WO 2021/033139

(56) References cited:
- US-A1- 2016 339 242
- US-A1- 2019 022 372
- US-A1- 2019 117 976
- US-B2- 10 016 601

## Description

### FIELD OF THE INVENTION

The present invention relates to methods of setting, calibrating, and confirming the reliability of, electrical parameters for treatments using electrical neurostimulation.

### BACKGROUND OF THE INVENTION

The present invention is defined in claim 1. Any embodiments disclosed herein involving neurostimulation do not form part of the scope of the invention, and are mentioned for illustrative purposes only. The disclosed methods and devices may be used for calibration of treatment parameters used in the stimulation of peripheral and cranial nerves, including calibration by the users of the neurostimulation treatments.

Peripheral and cranial nerves in the head region may be stimulated to treat various conditions such as chronic pain, migraine, tension headaches, cluster headaches, trigeminal neuralgia, occipital neuralgia, fibromyalgia, depression, post-traumatic stress disorder (PTSD), anxiety, stress, bipolar disorder, schizophrenia, obsessive compulsive disorder (OCD), insomnia, epilepsy, attention deficit disorder (ADD), attention deficit hyperactivity disorder (ADHD), Parkinson's disease, Alzheimer's disease, obesity, multiple sclerosis, stroke and traumatic brain injury (TBI). The anatomy of peripheral and cranial nerves in the head region, such as that of the occipital and trigeminal nerves, and their projections to brainstem regions such as the locus coeruleus and nucleus raphe magnus, as well as to higher brain regions such as the thalamus and anterior cingulate cortex, may be advantageous when stimulating these nerves for treatment of such conditions.

Neurostimulation of superficial nerves in the head region, such as the occipital, supraorbital, supratrochlear, zygomaticotemporal, and auriculotemporal nerve branches, can be applied either invasively or non-invasively. Due to the challenge of transferring current through the hair, stimulation of cephalic nerves which lie under hair covered regions such as the occipital nerve (greater, lesser and third occipital branches), is typically carried out using implanted or percutaneous nerve stimulators. Such devices include electrodes that are inserted under the scalp and thus bypass the high impedance barrier formed by the hair and scalp. However, implanted nerve stimulation remains an invasive and costly procedure with a high rate of complications including infection, bleeding or fluid collection under the skin, as well as hardware-related malfunctions such as migration and breakage of the implanted leads and pulse generator failure. Transdermal stimulation of cephalic nerves such as the occipital nerve branches using non-invasive techniques is likely to achieve similar clinical benefits to those of implanted stimulators without the risks and cost associated with an invasive procedure.

Transdermal neurostimulation treatments have the promise of being carried out by the subjects of such treatments in the comforts of their own personal surroundings. It is noted, however, that even transdermal treatments can require the user-specific and personalized setting of some treatment parameters such as, for example, the current intensities and pulse durations encountered during a treatment. Moreover, electrical stimulation directed at different parts of the user's head may require differentiated treatment parameters for effective stimulation of the different nerves involved. Treatment parameters are optimally set and calibrated so as to take into account both the efficacy of treatments and the comfort level of users undergoing the treatments.

There is therefore a recognized need for, and it would be highly advantageous to have, devices and methods for effective setting and calibration of neurostimulation treatment parameters that can operated effectively by both medical professionals and users of the treatment.

Systems, apparatuses and methods for delivery of neurostimulation treatments are disclosed in Applicant's patents US 9,433,774 and US 9,872,979, and in Applicant's co-pending US patent applications 15/510,067, filed on Sept. 16, 2015, and published as US 20170296121; 16/070,563, filed on Jan. 26, 2017, and published as US 20190022372; and 16/093,094, filed on April 9, 2017, and published as US 20190117976.

US 2019/022372 A1 relates to apparatus and methods for applying electrical stimulation to the head region.

### SUMMARY OF THE INVENTION

A method is disclosed, according to embodiments, for determining user-specific treatment parameter values for a multi-channel transdermal neurostimulation treatment applied at multiple locations on a user's scalp. The method comprises: (a) setting a first set of treatment parameter values for a first electrode-array in engagement with a first portion of the user's scalp for administering electrical pulses thereto, according to a first correlation that includes a first direct relationship between at least one parameter value of said first set of treatment parameter values and a corresponding at least one parameter value of a set of user-specific sensory-threshold parameter values; and (b) setting a second set of treatment parameter values for a second electrode-array in engagement with a second portion of the user's scalp for administering electrical pulses thereto, according to a second correlation that includes a second direct relationship between at least one parameter value of said second set of treatment parameter values and said at least one parameter value of said first set of treatment parameter values.

In some embodiments, each respective set of treatment parameter values can include values for at least two of the following: (i) an electrical charge parameter having values of total electric charge for a given time period, where the given time period is an integer multiple of an inverse of electrical pulse frequency, (ii) a current-intensity parameter, and (iii) a pulse duration parameter and a frequency parameter, or an arithmetic combination thereof.

In some embodiments, either (i) said first portion of the scalp is an anterior portion and said second portion of the scalp is a posterior portion, or (ii) said first portion of the scalp is a posterior portion and said second portion of the scalp is an anterior portion.

In some embodiments, said second direct relationship can be based in part on a relationship between respective electrode surface-areas of said first and second electrode arrays.

In some embodiments, values of the set of user-specific sensory-threshold parameter values are based on a first user input received during application of a series of electrical pulses to a single one of the first and second portions of the user's scalp.

In some embodiments, said first and second correlations can be linear relationships based on respective empirical correlations.

In some embodiments, it can be that according to said first direct relationship, an electrical charge parameter value of said first set of treatment parameter values is equal to an electrical charge parameter value of said set of user-specific sensory-threshold parameter values multiplied by A, where A is not less than 1.1 and not greater than 3.2. In some embodiments, it can be that A is not less than 1.4 and not greater than 2.0. In some embodiments, it can be that A is not less than 1.3 and not greater than 1.9.

In some embodiments, it can be that according to said second direct relationship, an electrical charge parameter value of said second set of treatment parameter values is equal to an electrical charge parameter value of said first set of treatment parameter values multiplied by B, where B is not less than 1.1 and not greater than 3.8. In some embodiments, it can be that B is not less than 1.4 and not greater than 3.4. In some embodiments, it can be that B is not less than 1.8 and not greater than 3.0.

In some embodiments, the method can additionally comprise: calibrating treatment thresholds, wherein the calibrating includes: (i) activating a transdermal neurostimulation calibration session using said first and second set of treatment parameter values as respective treatment parameter values applied to said first and second portions of the user's scalp, (ii) receiving a user-calibration input during said calibration session, and (iii) in response to receiving said user-calibration input, changing at least one value of said first and second set of treatment parameter values.

In some embodiments, said changing of said at least one value of said first and second set of treatment parameter values can include modifying at least one treatment parameter value that is not a current intensity.

In some embodiments, said changing can be constrained such that an electrical charge parameter value is constrained to being within a predetermined range. In some embodiments, said changing can be constrained such that the number of changes per calibration session is limited to a predetermined number of changes. In some embodiments, said changing can be constrained to a predetermined interval of time. In some embodiments, the time between receiving of the user-calibration input and completing the changing can be less than one second. In some embodiments, the time between receiving of the user-calibration input and completing the changing can be less than 500 msec. In some embodiments, the time between receiving of the user-calibration input and completing the changing can be less than 200 msec.

In some embodiments, the transdermal neurostimulation treatment can include anteriorly-applied electrical pulses targeting the trigeminal nerve and posteriorly-applied electrical pulses targeting the occipital nerve.

In some embodiments, said administering of electrical pulses can be by an apparatus including electrodes and mounted on the user's head, said apparatus comprising a headset.

In some embodiments, at least one of said first user input and said user-calibration input can be received via a computer software program running on an external user-operated device in electronic communication with said headset. In some such embodiments, it can be that (i) said headset comprises a on-headset user interface attached to said headset, and/or (b) at least one of said first user input and said user-calibration input is received via said on-headset user interface.

A method is disclosed, according to embodiments, for determining user-specific treatment parameter values for a multi-channel transdermal neurostimulation treatment applied at multiple locations on a user's scalp. The method comprises: (a) setting a first set of treatment parameter values for a first electrode-array in engagement with a first portion of the user's scalp for administering electrical pulses thereto; and (b) setting a second set of treatment parameter values for a second electrode-array in engagement with a second portion of the user's scalp for administering electrical pulses thereto, according to a second correlation that includes a direct relationship between at least one parameter value of said second set of treatment parameter values and said at least one parameter value of said first set of treatment parameter values.

In some embodiments, each respective set of treatment parameter values can include values for at least two of: (i) an electrical charge parameter having values of total electric charge for a given time period, where the given time period is an integer multiple of an inverse of electrical pulse frequency, (ii) a current-intensity parameter, and (iii) a pulse duration parameter and a frequency parameter, or an arithmetic combination thereof.

In some embodiments, it can be that either (i) said first portion of the scalp is an anterior portion and said second portion of the scalp is a posterior portion, or (ii) said first portion of the scalp is a posterior portion and said second portion of the scalp is an anterior portion.

In some embodiments, said direct relationship can be based in part on a relationship between respective electrode surface-areas of said first and second electrode arrays.

In some embodiments, wherein said correlation can be a linear relationship based on an empirical correlation.

In some embodiments, it can be that according to said direct relationship, an electrical charge parameter value of said second set of treatment parameter values is equal to an electrical charge parameter value of said first set of treatment parameter values multiplied by B, where B is not less than 1.1 and not greater than 3.8. In some embodiments, it can be that B is not less than 1.4 and not greater than 3.4. In some embodiments, it can be that B is not less than 1.8 and not greater than 3.0.

In some embodiments, the transdermal neurostimulation treatment can include anteriorly-applied electrical pulses targeting the trigeminal nerve and posteriorly-applied electrical pulses targeting the occipital nerve.

In some embodiments, said administering of electrical pulses can be by an apparatus including electrodes and mounted on the user's head, said apparatus comprising a headset.

A method is disclosed according to embodiments, for determining user-specific treatment parameters for a multi-channel transdermal neurostimulation treatment applied at multiple locations on a user's scalp. The method comprises: (a) providing, to the user's scalp, a first series of electrical pulses having electrical parameter values that increase at a first rate of increase, wherein said respective electrical parameter values comprise at least one of an electrical charge parameter value and a current-intensity value; (b) responsively to receiving a first user input indicating that the user has felt a dermal sensation related to the application of said first series of electrical pulses, defining, as a set of sensory-threshold parameter values, a plurality of respective electrical parameter values at which said first user input is received; (c) providing, to the user's scalp, a reliability-confirming series of electrical pulses having respective electrical parameter values that increase at a second rate of increase, wherein said respective electrical parameter values comprise at least one of an electrical charge parameter value and a current-intensity value, the providing being such that said reliability-confirming series is (i) paused before respective electrical parameter values reach said sensory-threshold parameter values, and (ii) subsequently resumed after a pause; (d) responsively to receiving, after said pause and at respective electrical parameter values within a predetermined range of values surrounding said sensory-threshold parameter values, a second user input indicating that the user has felt a dermal sensation related to the application of said reliability-confirming series of electrical pulses: confirming the reliability of said sensory-threshold parameter values; and (e) applying the multi-channel transdermal neurostimulation treatment using treatment parameter values based on said reliability-confirmed sensory-threshold parameter values.

In some embodiments, said treatment parameter values can include values for at least two of: (i) an electrical charge parameter having values of total electric charge for a given time period, where the given time period is an integer multiple of an inverse of electrical pulse frequency, (ii) a current-intensity parameter, and (iii) a pulse duration parameter and a frequency parameter, or an arithmetic combination thereof.

In some embodiments, it can be that said first series of electrical pulses is applied to a first portion of the user's scalp, and said reliability-confirming series of electrical pulses is applied to a second portion of the scalp that is different from the first portion. In some embodiments, it can be that said first series of electrical pulses and said reliability-confirming series of electrical pulses are both applied to a first portion of the user's scalp.

In some embodiments, it can be that either (i) said first portion of the scalp is an anterior portion and said second portion of the scalp is a posterior portion, or (ii) said first portion of the scalp is a posterior portion and said second portion of the scalp is an anterior portion.

In some embodiments, the transdermal neurostimulation treatment can include anteriorly-applied electrical pulses targeting the trigeminal nerve and posteriorly-applied electrical pulses targeting the occipital nerve.

In some embodiments, said providing of said first series of electrical pulses and said providing of said reliability-confirming series of electrical pulses can be by an apparatus including electrodes and mounted on the user's head, said apparatus comprising a headset.

In some embodiments, at least one of said first user input and said second input can be received via a computer software program running on an external user-operated device in electronic communication with said headset.

In some embodiments, it can be that (i) said headset comprises a on-headset user interface attached to said headset, and (ii) at least one of said first user input and said second user input is received via said on-headset user interface.

According to embodiments, a non-transitory computer-readable medium having stored therein program instructions, which when executed by one or more processors, cause the one or more processors to carry out any of the steps of any of the methods disclosed herein.

According to embodiments, a user input device for use during a transdermal neurostimulation treatment comprises a user interface; one or more processors; and a non-transitory computer-readable medium having stored therein program instructions, which when executed by said one or more processors of the user input device, cause said one or more processors to carry out any of the steps of any of the methods disclosed herein. In some embodiments, the user input device can be in electronic communication with an apparatus comprising a headset and a plurality of electrodes configured to deliver electric pulses to a user's scalp.

### BRIEF DESCRIPTION OF THE FIGURES

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice. Throughout the drawings, like-referenced characters are used to designate like functionalities, but not necessarily identical elements.

In the drawings:
Fig. 1 is a schematic block diagram of an embodiment of a system for neurostimulation according to an embodiment of the teachings herein.
Fig. 2 provides a schematic, perspective view illustration of a donned, inventive headset adapted to communicate with a remote-control unit, mobile phone, and computer.
Fig. 3 is a perspective view schematic illustration of an embodiment of the inventive system of Figure 1A in the form of a headset according to the teachings herein.
Figs. 4-13 show flowcharts of method building blocks comprising method steps for defining, setting, testing, confirming, calibrating and applying parameters for transdermal neurostimulation, according to embodiments.
Figs. 14-19 show flowcharts of methods which various method steps and method building blocks of Figs. 4-13, according to embodiments.

### DETAILED DESCRIPTION

Systems and methods are described herein that apply electrical stimulation to the head region, for stimulation of peripheral and/or cranial nerves, transcranial stimulation of brain regions, and sensing of body parameters, while monitoring the tissue and adapting the electrical stimulation signal to the user's characteristics and to changes over time. The systems and methods ensure effective electrical stimulation while avoiding damage to scalp tissue as well as discomfort to the user, and operate in a safe and robust fashion.

The methods may be applied using a head mounted construction serving as a platform for applying electrical stimulation in accordance with the inventive methods to treat various conditions such as migraine, tension type headaches, cluster headaches, trigeminal neuralgia, occipital neuralgia, chronic pain, fibromyalgia, stress, depression, post-traumatic stress disorder (PTSD), anxiety, obsessive compulsive disorder (OCD), insomnia, epilepsy, attention deficit hyperactivity disorder (ADHD), Parkinson's disease, Alzheimer's disease, obesity, multiple sclerosis, traumatic brain injury (TBI) and stroke.

The term 'user' as employed herein means a person who receives or is going to receive transdermal stimulation treatment using electrical stimulation. Electrical stimulation of a pure sensory nerve elicits radiation of a paresthesia sensation along the distribution of the nerve in response to applied electrical stimulation.

This disclosure deals with 'dermal sensation' that a user may experience in response to the application of transdermal electrical neurostimulation, and may include one or more of tingling, pricking, chilling, burning, or numbness; such dermal sensation may be equivalent to the sensation of paresthesia.

A minimum threshold at which a user feels or acknowledges feeling such a dermal sensation or paresthesia is called a 'sensory threshold'; a sensory threshold may be particular to a user at a given time under given circumstances and conditions.

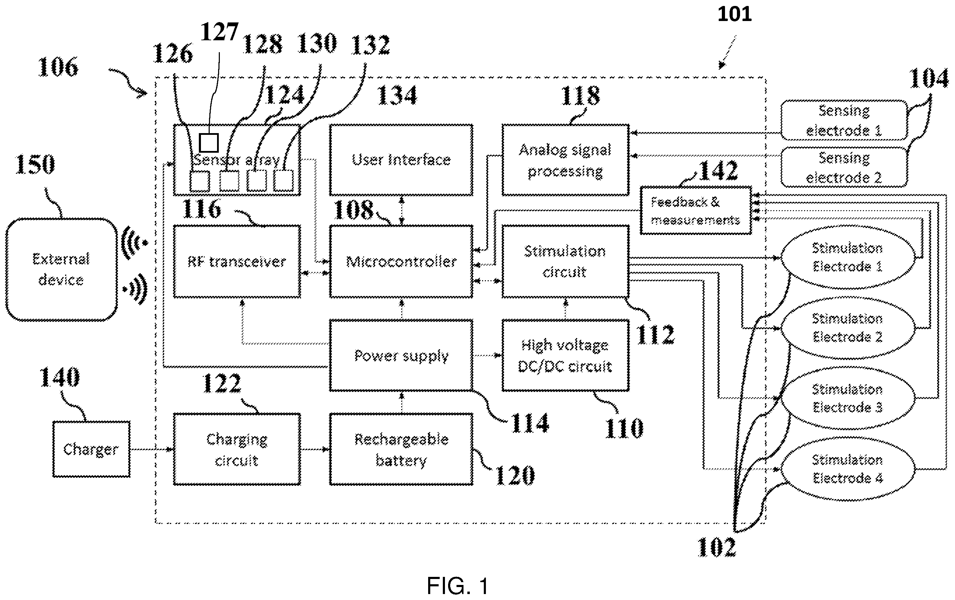

Reference is now made to Fig. 1, which is a schematic block diagram of an embodiment of an exemplary system for neurostimulation according to an embodiment of the teachings herein.

As seen, a system **101** for neurostimulation may include at least two stimulating electrodes **102,** and in some embodiments may further include at least two sensing electrodes **104,** both the electrodes **102** and **104** functionally associated with an electronic circuit **106.** The stimulating electrodes **102** are adapted to engage the skin of the user's scalp so as to deliver current thereto as described hereinbelow. In some embodiments, one or more of sensing electrodes **104** may be adapted to engage the skin of the user, and may be configured to sense at least one electrical parameter of a body portion of the user, such as, for example, electroencephalogram (EEG), skin conductance response (SCR), impedance plethysmograph (IPG), electromyograph (EMG), and the like.

According to features of the teachings herein, system **101,** and specifically the electronic circuit **106,** may be suited for applying transcranial electrical stimulation using suitable methods such as Transcranial Direct Current Stimulation (tDCS), Transcranial Alternating Current Stimulation (tACS), and Transcranial Random Noise Stimulation (tRNS). Insofar as a treatment or application of electric stimulation described herein relates to a user's head or scalp, then the terms 'transdermal' and 'transcranial' may be used interchangeably. The term 'scalp' as used herein should be understood in the broadest sense of the term, and especially as being inclusive of the skin of the forehead, i.e., that part of a user's 'face' which is above the user's eyes.

As seen, the electronic circuit **106** may include any one or more of a microcontroller **108,** a high voltage circuit **110,** a stimulation circuit **112,** an internal power supply **114,** a radio-frequency (RF) transceiver **116,** an analog signal processing circuit **118,** a rechargeable battery **120** electrically associated with a charging circuit **122,** a sensor array **124** including one or more of an accelerometer **126,** a temperature sensor **128,** a pressure sensor **130,** and a humidity sensor **132,** and a user interface **134.**

In some embodiments, the electronic circuit **106** may be electrically associated with and powered by rechargeable battery **120** that is electrically connected to internal power supply **114.** In some embodiments, the internal power supply **114** provides power to high voltage circuit **110,** which in turn is electrically connected to stimulation circuit **112.** The charging circuit **122** is electrically associated with rechargeable battery **120,** and may interface with an external power supply, such as a charger **140.** The high voltage circuit **110** provides to stimulation circuit **112** current with voltage in the range of 1 to 150 V. In some embodiments, the rechargeable battery **120** may be replaced by a consumable battery configured to operate in the same ranges of voltage and current.

In some embodiments, the stimulation circuit **112** receives information and/or commands from the microcontroller **108.** The stimulation circuit **112** is configured to provide electrical stimulation pulses to the user's nerve tissues via the stimulation electrodes **102.**

The stimulation circuit **112** may be configured to produce biphasic, charged balanced electrical pulses, mono-phasic electrical pulses, and/or direct current stimulation.

According to still further features of the described embodiments, the stimulation circuit **112** may be configured to produce electrical stimulation within an intensity range of 0-60mA, 0-40mA, 0-20m, or 0-15mA.

According to still further features of the described embodiments, the stimulation circuit **112** may be configured to produce stimulation pulses with a duration of 10-1000 µsec, 50-600 µsec, 100-500 µsec.

According to still further features of the described embodiments, the stimulation circuit **112** may be configured to produce stimulation pulses at a frequency of 1-20,000Hz, 1-10,000Hz, 1-500Hz, 10-300Hz, 10-250Hz, 20-180Hz, 30-180Hz or 40-100Hz.

In some embodiments, electronic circuit **106** may include two or more high voltage circuits (not shown) similar to circuit **112,** each high voltage circuit providing current at a voltage of 1-150V, 1-120V, 1-100V, to at least two of stimulation electrodes **102.** In some embodiments, electronic circuit **106** may include at least two isolated output channels (not shown), each output channel providing output to at least two of stimulation electrodes **102.**

In some embodiments, the electronic circuit **106** also includes a feedback & measurements circuit **142,** which collects voltage or current level information from the stimulation electrodes **102,** and provides the collected information to the microcontroller **108.** The microcontroller **108** uses the provided feedback to monitor and control the voltage and current levels in stimulation electrodes **102** via stimulation circuit **112.** In some embodiments, the microcontroller **108** may alert the user, for example by providing an audible or tactile indication, or may halt the provision of current for stimulation in the case of an emergency or of incorrect function of the system.

In some embodiments, the microcontroller **108** may instruct the stimulation circuit **112** to output electrical current in various patterns and/or for various periods of time, and/or may instruct the stimulation circuit **112** with regards to various stimulation parameters, such as the current amplitude, pulse frequency, phase duration, and amplitude of the current output by the stimulation circuit.

In some embodiments, the microcontroller **108** may instruct the stimulation circuit **112** to provide an output signal having a different pattern for each of a plurality of activated pairs of electrodes. For example, the stimulation circuit **112** may stimulate one pair of electrodes at a pulse frequency of 50Hz and a phase duration of 300µsec and another pair of electrodes at a pulse frequency of 100Hz and a phase duration of 200µsec. In some embodiments, at any given time the microcontroller **108** may activate only one pair of electrodes, may activate a combination of electrodes, and/or may activate several electrodes simultaneously, sequentially, or alternately.

In some embodiments, some electrodes **102** may provide as output an alternating current signal, whereas other electrodes **102** may provide as output a direct current. In some embodiments, at least two electrodes **102** may alternate the type of current provided as output between alternating current and direct current.

In some embodiments, during direct current stimulation in which excitation of a certain region of the brain is determined based on the polarity of an electrode which is positioned above that region of the brain, at least one electrode **102** may be assigned by the microcontroller **108** to be the anode, or positively charged electrode, and at least one other electrode **102** may be assigned to be the cathode, or negatively charged electrode.

In some embodiments, stimulation patterns determined by or assigned by the microcontroller **108,** as well as feedback data received from electrodes **102** and/or from sensing electrodes **104** may be stored in the microcontroller **108** or in a volatile or non-volatile memory (not shown) associated therewith. In some embodiments, the stored stimulation patterns may be used to create a personalized neurostimulation treatment protocol.

In some embodiments, electronic circuit **106** may be configured to receive analog signal input, such as electroencephalogram (EEG) signals, skin conductance response (SCR) signals, impedance plethysmograph (IPG) signals, electromyograph (EMG) signals, or other bio-signals, from one or more sensors, such as sensing electrodes **104,** which bio-signals may be indicative of the impedance of the tissue receiving the neurostimulation signal, the charge provided to the tissue, or the like. The analog signal input received from sensing electrodes **104** may be processed by analog signal processing circuit **118,** and may be transferred therefrom to microcontroller **108.** In some embodiments, electronic circuit **106** may be configured to receive digital, analog, or other input from additional sensors adapted to sense the vicinity of the user or characteristics thereof. In some embodiments, one or more stimulation parameters may be altered by the microcontroller **108** due to inputs received from one or more of the additional sensors, as described hereinbelow.

In some embodiments, accelerometer **126,** or any other suitable orientation sensor, may be configured to sense the angular position of the user's head or of an apparatus embodying system **101** (and particularly portions thereof engaging the user's head), and thereby may enable microcontroller **108** to identify a change in the user's and/or system's conditions and to adjust or adapt the pulse provided by stimulating electrodes **102.** For example, a change in the position of the user may result in a change in the pressure applied to the electrodes, thus changing how close the electrodes are to the user's skin and consequently changing the impedance in the system and requiring adaptation of the pulse applied to the tissue via the electrodes, as described hereinbelow. In some embodiments, a vibrating structure microelectromechanical systems (MEMS) gyroscope **127** can be configured to provide angular motion information additional to motion and orientation information provided by the accelerometer **126.**

In some embodiments, temperature sensor **128** may be configured to sense a temperature in the vicinity of the system **101** or of the stimulating electrodes **102,** and thereby may enable microcontroller **108** to identify a change in the user's and/or system's conditions and to adjust or adapt the pulse provided by stimulating electrodes **102.** For example, an increase in the temperature in the vicinity of the user or of the electrodes **102** may result in more rapid dehydration of the electrodes or of conducting material applied thereto, thus increasing the impedance in the system and requiring adaptation of the pulse applied to the tissue via the electrodes, as described hereinbelow.

In some embodiments, pressure sensor **130** may be configured to sense pressure applied to the user's head in the vicinity of electrodes **102** or pressure applied directly to electrodes **102,** and thereby may enable microcontroller **108** to identify a change in the user's and/or system's conditions and to adjust or adapt the pulse provided by stimulating electrodes **102.** For example, an increase in the amount of pressure applied to electrodes **102** pushing them towards the user's scalp is expected to reduce the distance between the electrodes and the scalp, and in some cases the distance between the electrode and the target nerve, thereby reducing the impedance in the system and requiring, or allowing, adaptation of the pulse applied to the tissue via the electrodes.

In some embodiments, humidity sensor **132** may be configured to sense a humidity or moisture level in the vicinity of the system **101** or of the stimulating electrodes **102,** and thereby may enable microcontroller **108** to identify a change in the user's and/or system's conditions and to adjust or adapt the pulse provided by stimulating electrodes **102.**

In some embodiments, user interface **134** may be configured to receive from the user an indication of the sensation the user is feeling, such as an indication of pain, an indication of discomfort, or an indication of decreased, or no, paresthesia (dermal sensation). Such an indication from the user of a change in the sensation the user feels may enable microcontroller **108** to adjust or adapt the pulse provided by stimulating electrodes **102,** or to assess sensory and treatment thresholds and user-calibration of thresholds, as will be described elsewhere in this disclosure.

In some embodiments, RF transceiver **116** may enable the microcontroller **108** to communicate with an interface of an external device **150,** such as a mobile phone, a tablet, a computer, or a cloud-based database, by way of radio frequency. The RF transceiver **116** may transmit digital information to and may receive digital information from the microcontroller **108,** for example for personalization of the neurostimulation treatment provided by system **101.** RF transceiver **116** may be operable to transmit/receive in any of the wireless communications protocols known in the art, including, but not exhaustively, Wi-fi, WLAN, PDA, VoIP, and Bluetooth.

The interface of device **150** may comprise a software application that may be downloadable from a readily accessible resource, such as from the Internet. The interface may provide to a user thereof an indication, for example by way of a display, of the status of the system **101,** including, for example, information relating to active stimulation channels, stimulation intensity, active program, treatment time, battery status, and RF communication status, as well as various alerts such as alerts relating to electrode contact quality and to proper or improper system alignment on the head. Additionally, the interface may provide to the user, for example by way of a display, usage logs and/or reports, such as information relating to daily stimulation time, stimulation parameters which were used during stimulation, and treatment programs which were used. The interface may also display, or otherwise provide, to the user raw or processed information received from sensors included in or associated with the apparatus.

In some embodiments, the system may be controlled remotely via the interface of external device **150.** For example, the external interface may enable a user thereof to activate or turn off the system, start or pause stimulation, adjust the stimulation intensity for one or more channels, and select a treatment program. In some embodiments, information collected by the microprocessor **108** may be transmitted, via the external interface, to a remote location, such as a cloud-based portal, where the information may be stored or may be analyzed and/or monitored.

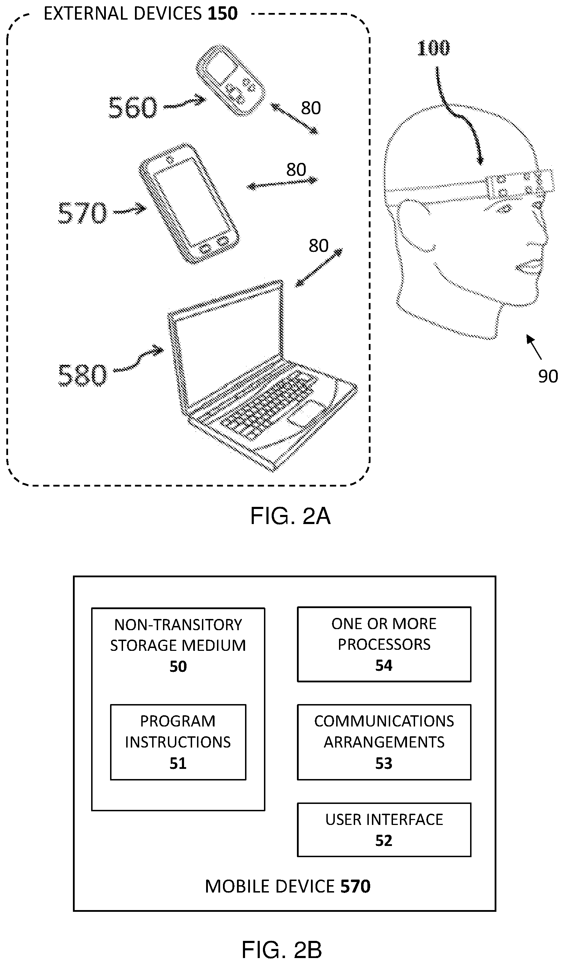

We now refer to Fig. 2A, which illustrates several components of a neurostimulation treatment system. A headset **100** is mounted on the head **90** of a user. In some embodiments, headset **100** may be configured to communicate wirelessly with one or more external devices **150** using communications channel(s) **80.** Any communications channel **80** may utilize any combination of direct device-to-device communications (e.g., by wi-fi or Bluetooth or IR or any other wireless device-to-device communication or wired communication) and indirect communications (e.g., routed through servers and/or routers and/or other local network devices and Internet devices).

An example of an external device **150** is remote control **560,** which may be used by the user to send commands and other inputs to headset **100.** Remote control **560** may also present various visual and audio indications for the user regarding the status of headset **100.** Additionally or alternatively, headset **100** may be configured to wirelessly communicate with a mobile phone **570.** The mobile phone interface may be used to present various data sent wirelessly by headset **100,** for example, visual and audio indications regarding the status of headset **100** and usage logs. As shown in the block diagram of an exemplary handheld device such as a mobile phone **570** in Fig. 2B, mobile phone **570** includes a non-transitory computer-readable storage medium **50** on which are stored program instructions **51** for execution by the one or more processors **54** of the mobile phone **570.** The program instructions **51** may cause the mobile phone **570** and/or the headset **100** to control a neurostimulation treatment session, including, for example, to determine treatment protocol parameters before a treatment session, to capture user instructions and other inputs, and/or to determine treatment protocol parameters. The mobile phone **570** of Fig. 2B additionally includes communications arrangements **53** for communicating with headset **100** (inter alia), and a user interface **52** which may include a touch screen. The user interface **52** can be used for any manner of interactions between user and the software program comprising program instructions **51,** and the user interface **52** may be used to present various data sent wirelessly by headset **100,** such as visual and audio indications regarding the status of headset **100** and usage logs.

Still referring to Fig. 2A, headset **100** may additionally or alternatively be configured to wirelessly communicate with laptop/PC **580.** Like mobile phone **570,** Laptop/PC **580** may be configured with a non-transitory computer-readable storage medium on which are stored program instructions **51.**

Any one or more of the illustrated 'user input devices' **150** of Fig. 2A (mobile phone **570,** remote control **560,** and PC/laptop **580)** may be used interchangeably in carrying out the methods disclosed herein. Headsets **100** suitable for carrying out the methods disclosed herein include any of the headsets disclosed in Applicant's patents US 9,433,774 and US 9,872,979, and in Applicant's co-pending US patent applications 15/510,067, filed on Sept. 16, 2015, and published as US 20170296121; 16/070,563, filed on Jan. 26, 2017, and published as US 20190022372; and 16/093,094, filed on April 9, 2017, and published as US 20190117976.

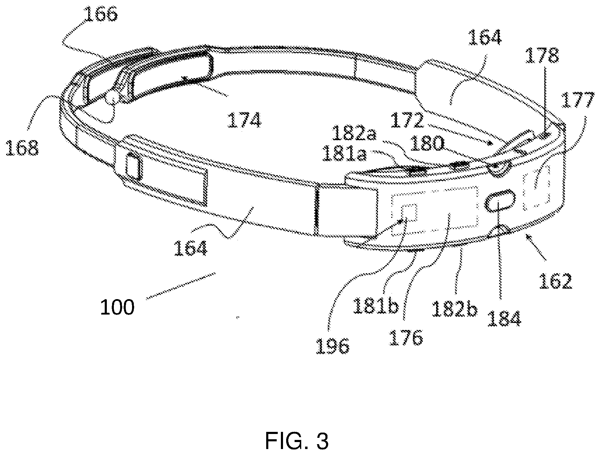

Reference is now made to Fig. 3, which is a perspective view schematic illustration of a non-limiting exemplary embodiment of a headset **100** implementing the system **101** of Fig. 1. As seen, a headset **100** may be configured to include an anterior member **162** connected to a pair of flexible arm members **164,** which may also be called interim members, each terminating in a posterior member **166.** Anterior member **162,** flexible arm members **164,** and posterior members **166** together form the headset's body. In some embodiments, electrode systems **172** may comprise anterior electrodes adapted to be located at the supraorbital region of the head over the trigeminal nerve branches for stimulation thereof, or may be electrodes suitable for transcranial stimulation of the frontal and prefrontal region of the brain. In some embodiments, electrode systems **174** may comprise posterior electrodes adapted to be located at the occipital region of the head over the occipital nerve branches for stimulation thereof, or may be electrodes suitable for transcranial stimulation of the occipital region of the brain.

It will be appreciated that headset **100** may include additional electrodes having similar structure and/or functionality to those of electrode systems **172** and **174.** It is further appreciated that electrode systems **172** and/or **174** may be obviated, or moved to other locations on headset **100,** as suitable for stimulating specific nerves or nerve sets, or specific brain regions. For example, electrode systems **174** may be moved to be along the flexible arm members **164.** As another example, the headset **100** may include only a single pair of electrode systems located on arm members **164,** which electrodes may be configured to be positioned, when the headset is donned, under the hair, while electrode systems **172** and **174** may be obviated. In some embodiments, electrodes can be directed to stimulating nerves located in lateral regions of the brain, additionally or alternatively to stimulation of anterior and/or posterior nerves.

Anterior member **162** may be configured to contain an electronic circuit **176,** similar to electronic circuit **106** of Figure 1, which may be configured to be electrically coupled by conductive wires (not shown) to a power source **177,** such as a battery similar to battery **120** of Figure 1, and to electrodes systems **172** and **174.** In some embodiments, at least a portion of the conductive wires extends to posterior electrode systems **174** via arm members **164.**

In some embodiments, the electronic circuit **176** and/or the battery **177** may be external to headset **100,** and/or may communicate remotely with headset **100.**

As discussed hereinabove with reference to Figure 1, the electronic circuit **176** may include a stimulation circuit, a microprocessor, a charging circuit and a user interface.

In some embodiments, headset **100** may be configured to connect to an external electronic circuit and/or stimulation circuit, and thereby to transfer electrical current from an external stimulator to the electrode systems **172** and/or **174.** In some embodiments, headset **100** may be configured to connect to at least one external electrode that may be located at various areas of the body. In some embodiments, headset **100** may be configured to connect to an external electronic circuit and processor in order to transfer signals from sensors disposed on the headset **100** to the external processor.

In some embodiments, battery **177** may be disposed within anterior member **162,** and may be recharged by plugging a charger into charging port **178** located, according to certain embodiments, on anterior member **162.**

Anterior member **162** may also be configured to include, on an external surface thereof, user controls and interface **180,** which may be similar to user interface **134** of Figure 1. That said, in some embodiments, other portions of headset **100,** such as posterior members **166** or arms **164,** may be configured to include user interface **180.** Some of the functionalities of on-board (i.e., attached or installed, either permanently or removably) user interface **180, 134** may overlap with those of the user interface **52** of external device **150,** such as, for example, enabling the receiving of user inputs to control a neurostimulation treatment or to set one or more treatment-related parameters. In some embodiments, a user may use both an on-headset user interface **180** and an on-device user interface **52,** while in other embodiments only one of the two user interfaces **180, 52** is available to a user.

Electronic circuit **176** and user interface **180** may be configured to control and/or activate electrodes included in headset **100.** In some embodiments, user interface **180** is configured to control and/or activate at least two, and in some embodiments more than two, pairs of electrodes. As such, in some embodiments, the stimulation circuit and/or user interface **180** are configured to enable activation of a specific electrode or of a specific pair, or channel, of electrodes, as well as adjustment of the intensity of current supplied by the activated electrodes or of other stimulation parameters of the activated electrodes and provision of user indications such as a user indication of pain, a user indication of discomfort, or a user indication of reduced or increased paresthesia. In some embodiments, any subset of the electrodes may be activated simultaneously, and in some embodiments specific subsets are predefined, for example during manufacture of the electronic circuit **176.** In some such embodiments, user interface **180** enables control not only of a specific electrode or of a specific channel, but also of activated subsets of the electrodes.

In some embodiments, user controls and interface **180** includes a pair of anterior intensity buttons **181a** and **181b** for respectively increasing and decreasing the intensity of stimulation provided by anterior electrode systems **172,** and a pair of posterior intensity buttons **182a** and **182b** for respectively increasing and decreasing the intensity of stimulation provided by posterior electrode systems **174.** It will be appreciated that user control and interface **180** may include similar intensity buttons for each electrode included in the headset **100.**

The user controls and interface **180** may further include a mode changing button **184** for activating and disabling the electronic circuit **176,** as well as for changing between modes of operation of headset **100.** For example, headset **100** may have multiple preset modes of operation, such as a sleep mode, a maintenance mode, and a treatment mode, and repeated operation of button **184** may switch between these modes, in addition to turning the headset on and off.

A user indication button, for example allowing the user to provide a user indication of pain, discomfort, or reduced paresthesia, may form part of user controls and interface **180** and may be disposed on an exterior surface of anterior member **162.**

In some embodiments, the user controls and interface **180** may further include an audio element (not shown), such as a speaker or buzzer, for providing to the user an audible indication of use of the headset **180,** such as an indication of activation of the headset, shutting down of the headset, pressing a button on interface **180,** changing the stimulation mode, and the like.

As explained hereinabove, the electronic circuit and the user interface are configured to control and/or activate electrodes included in headset **100.** In some embodiments, the user interface is configured to control and/or activate at least two, and in some embodiments more than two, pairs of electrodes. As such, in some embodiments, the stimulation circuit and/or the user interface are configured to enable activation of a specific electrode or of a specific pair, or channel, of electrodes, as well as adjustment of the intensity of current supplied by the activated electrodes or of other stimulation parameters of the activated electrodes. In some embodiments, any subset of the electrodes may be activated simultaneously, and in some embodiments specific subsets are predefined, for example during manufacture of the electronic circuit. In some such embodiments, the user interface enables control not only of a specific electrode or of a specific channel, but also of activated subsets of the electrodes.

In some embodiments, electronic circuit **176** includes a transceiver **196,** similar to transceiver **116** of Figure 1, which transceiver is configured to remotely communicate with external device **150.**

According to embodiments of the present invention, methods for setting treatment parameters for a neurostimulation treatment session can include determining a sensory threshold based on a series of electrical stimulation pulses generated by a first plurality of electrodes, testing the reliability of the determined sensory threshold, and confirming the reliability of the determined sensory threshold. At any point in this process, i.e., after the determining, the testing, or the confirming, a first treatment threshold (recommended initial parameter) can be set based on the sensory threshold. A second treatment threshold can be set based on the first treatment threshold. The setting of a second treatment threshold based on a first treatment threshold can be based on a direct relationship between the two, for example a direct relationship derived empirically. The sensory threshold can be a treatment parameter or multiple treatment parameters at which a user indicates that he feels a paresthesia (or, more generally, any dermal sensation) related to a series of electrical stimulation pulses provided at current intensity that is monotonically increasing.

Treatment parameters, as the term is used herein can mean any (or all) of the following:
- an electrical charge parameter. An electrical charge parameter can have values of total electric charge for a given time period; the given time period is typically an integer multiple of an inverse of electrical pulse frequency (which can be called the 'period' of the pulses. As an illustrative example, if the frequency of pulses is 50 Hz, then the inverse of the frequency is 20 milliseconds (msec), and the given time period chosen might be 1 sec. For more granular resolution, the given time period can be as short as a single 'period', or 20 msec in this case. The units of the electrical charge parameter can be in coulombs (a smaller division such as milli-coulombs) per second, and can be calculated by averaging or integrating delivered current (e.g., in milli-amperes) over the given time period.
- a current-intensity parameter, for example a peak current or average current or steady-state current of an electrical pulse.
- a pulse duration parameter and a frequency parameter, or an arithmetic combination thereof. A pulse duration, also called pulse width, is typically measured in microseconds (µsec). It can be parameterized in conjunction with the period (inverse of the frequency), for example to derive a proportion of each period in which a pulse is being applied. As an example, a pulse duration (pulse width) of 500 µsec with a 50Hz frequency means that the pulse is being applied 0.5 msec / 20 msec of the period, or 2.5% of the time.

It can be desirable to use any two or more of the foregoing electrical parameters to characterize a series of pulses. For example: current intensity and electrical charge, or current intensity and a ratio of pulse duration to an inverse of the frequency, and electrical charge and a ratio of pulse duration to an inverse of the frequency are three exemplary sets of electrical parameters that can be used to characterize a series of pulses. A skilled artisan will understand that any of the foregoing sets can be used to calculate other parameters as well: for example, if average current intensity of a pulse and the electrical charge per period are known, then the proportion of the time (out of each period) that a pulse is being applied can be calculated.

In some instances, current intensity is used herein to illustrate the use of electrical parameters in the various embodiments, for example the use of monotonically increase current intensity in a method for setting, testing, confirming or calibrating a sensory threshold. In such instances, the illustrative use of current intensity is not meant to be limiting, and, where applicable, other electrical parameters or sets of electrical parameters can be substituted for current intensity.

A sensory threshold can be measured, in an exemplary embodiment, using electrical stimulation pulses generated by anteriorly placed electrodes, e.g., electrodes **172** of Fig. 3. In other embodiments electrodes can additionally or alternatively be laterally placed, although this is not shown in Fig. 3.

The first treatment threshold, in such an embodiment, can thus be related to anteriorly-directed pulses of a treatment protocol, and can be calculated from the sensory threshold using a first empirically-developed direct relationship. The second treatment threshold in such an embodiment can be related to posteriorly-directed pulses of a treatment protocol and can be calculated from the first treatment threshold using a second empirically-developed direct relationship. In some other embodiments, the first treatment threshold can be related to posteriorly-directed pulses of a treatment protocol and the second treatment threshold to anteriorly-directed pulses of the protocol. In still other embodiments, the first and second treatment thresholds can relate, respectively, to anteriorly directed pulses and laterally directed pulses; to posteriorly directed pulses and laterally directed pulses; to laterally directed pulses and anteriorly directed pulses; to laterally directed pulses and posteriorly directed pulses; or to laterally directed pulses on opposing sides of the user's head. The first and second direct relationships can be relationships that have been derived empirically on the basis of treatment thresholds known to be in an effective range with current intensity higher than the minimum required to achieve a noticeable paresthesia and lower than a threshold that causes pain or excessive discomfort for a user. Any of the aforementioned steps can be performed using a head-mounted apparatus, including any of the headsets **100** discussed herein. According to preferred embodiments, prior to a treatment session, a user can 'calibrate' the initial treatment parameters, i.e., the first and second treatment thresholds set in accordance with the foregoing discussion, by increasing or decreasing a treatment parameter (e.g., current intensity or pulse frequency) based on, inter alia, user comfort. Embodiments of the method can be carried out by executing program instructions **51** stored on an external device **150** such as a mobile phone **570,** the executing of the program instructions **51** being by one or more processors of the device **150,** as will be described later in this disclosure.

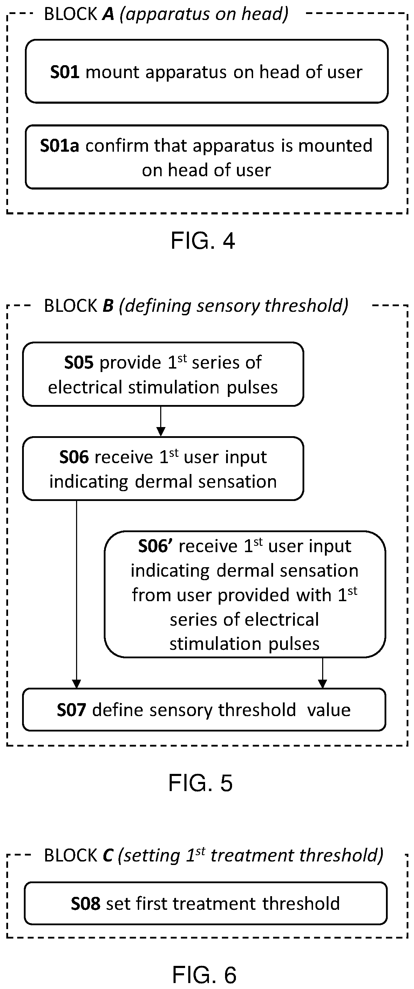

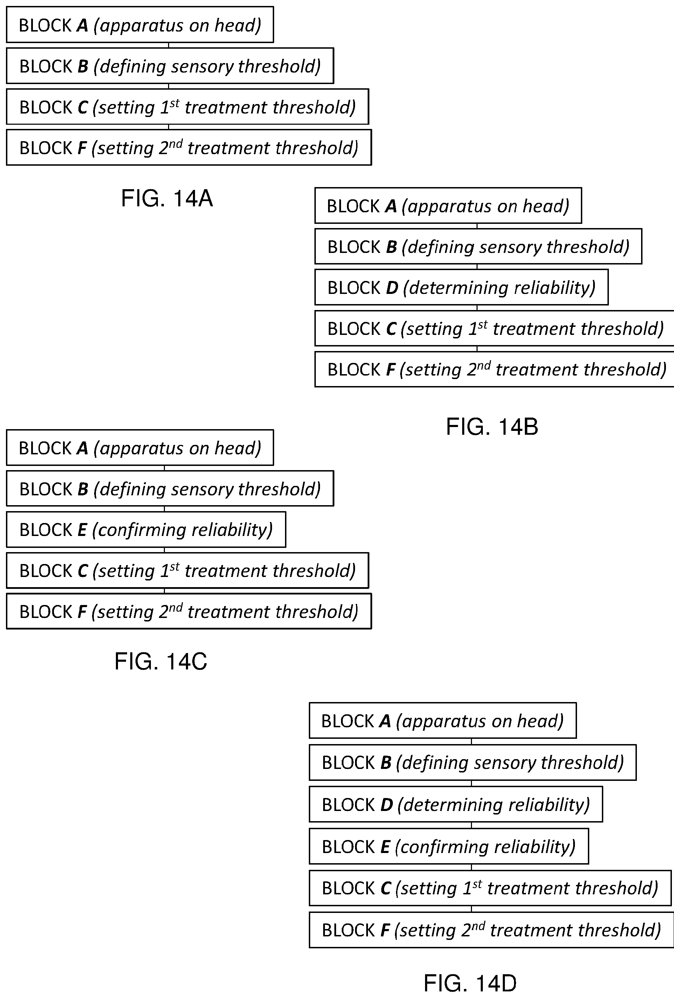

Reference is now made to Figs. 4-9, where flowcharts of methods and method 'building-blocks' relating to, inter alia, defining and setting user-specific sensory and treatment thresholds are shown in accordance with various embodiments. As seen, each building-block - a grouping of one or more method steps - is labeled with a block code (e.g., BLOCK A, etc.) so as to simplify later figures discussed hereinbelow that present different combinations of the various method building-blocks. Each building block includes a brief text description (e.g., BLOCK *A (apparatus on head))* for purposes of easy and convenient identification in the flowcharts of Figs. 14-19, where blocks are combined in various combinations. However, these brief text descriptions alongside the block designations are provided solely to enable easier comprehension of Figs. 14-19 and are not intended to delineate or limit in any way the scope of the corresponding method steps and building blocks. Any of the individual building blocks can constitute a method according to the disclosure, and any combination of building blocks can jointly constitute a method according to the disclosure, regardless of whether such methods are explicitly illustrated and/or described herein.

Referring now to Fig. 4, 'Block ***A** (apparatus on head) '* comprises Step S01, which includes mounting, on the head of a user, an apparatus comprising electrodes configured to provide electrical stimulation pulses, of the type used in transdermal neurostimulation treatments. In some embodiments, the apparatus comprises the electrodes and wires connecting the electrodes to a power source. In some embodiments, the apparatus additionally comprises control circuitry. The apparatus can additionally comprise an element for holding the electrodes in place in contact with the user's scalp, such as a headband or stretchable band. In some embodiments, the apparatus can be a headset such as, for example, any of the headsets **100** discussed previously in this disclosure. When the term headset is used hereinbelow, the term should be understood to include any apparatus comprising electrodes without necessarily having some or all of the other accoutrements of a full-featured headset such as those shown in the example of headset **100** of Fig. 3. In some embodiments, Block A additionally or alternatively comprises Step **S01a,** which includes confirming that such an apparatus is mounted on the head of a user. As an example, if a user has not yet donned the apparatus, then most likely Step **S01** will be performed. In contrast, if a user has already donned the apparatus, then most likely Step **S01a** will be performed. Of course, it is possible to perform both Step **S01** (in which the user dons the apparatus) and Step **S01a** (in which the donning of the apparatus is confirmed). In some embodiments, the confirming is included in program instructions **51** carried out by one or more processors **54** of a user input device such as a mobile phone **570.** In some such embodiments, the confirming can include receiving confirming information from a sensor such as, for example sensing electrodes **104.** In other such embodiments, the confirming can include receiving a user input on a user interface **52** of mobile device **570.** The confirming can additionally include soliciting a user input on user interface **52** prior to receiving the user input. Additionally, or alternatively, the confirming can include receiving a user input via on-board interface **180.**

Block ***B** (defining sensory threshold),* as illustrated in Fig. 5, comprises a group of method steps for determining a sensory threshold for a specific user.

In Step **S05,** a series of electrical stimulation pulses is provided by a set of stimulating electrodes **102** - in some embodiments, the set of stimulating electrodes **102** includes a plurality of anteriorly placed electrodes. The series preferably begins with faint pulses, i.e., pulses that are below a chosen electrical parameter such as a current intensity threshold or an 'electrical charge over time threshold' where the user experiences any dermal sensations related to the pulses. The pulses then increase monotonically in the chosen electrical parameter(s) as the series progresses. In some embodiments, other parameters of the pulses (e.g., pulse width, phase width, and frequency) remain unchanged.

Step **S06** comprises receiving a user input indicating that the user has experienced a dermal sensation, e.g., paresthesia, related to the series of pulses. Alternative Step **S06'** comprises receiving a user input indicating that the user has experienced, during a series of electrical stimulation pulses of increasing current intensity, or electrical charge over time, applied by a plurality of stimulating electrodes **102** to specific area or areas (e.g., anterior region) of the user's scalp, a dermal sensation related to the series of pulses. Step **S06'** can be included in program instructions **51** carried out by one or more processors **54** of a user input device such as a mobile phone **570.** The user input of Step **S06** or Step **S06'** is preferably received via a user interface **52** of mobile device **570** and/or on-board interface **180.**

Note: Wherever reference numbers used in this disclosure include an apostrophe after the number, e.g., S06', it is to indicate an alternative version of a step - such as S06 - that is specifically meant to be included in program instructions carried out by one or more processors of a user input device such as a mobile phone. In each case the technical concept is the same for each of the corresponding steps in a pair such as, for example, S06 and S06',.

In response to the receiving of the first user input of Step **S06** or Step **S06',** a sensory threshold is defined in Step **S07,** where the sensory threshold for any electrical parameter or set of electrical parameters is equal to the respective parameter values at which the user input is received. In other words, the sensory threshold corresponds to the electrical parameters at which the user experiences noticeable dermal sensation (e.g., paresthesia) from the series of electrical stimulation pulses.

Referring to Fig. 6, Block *C (setting 1^{st} treatment threshold)* comprises Step **S08,** which includes setting a first treatment threshold based on a relationship between the first treatment threshold and the sensory threshold. The relationship, in preferred embodiments, is a direct relationship derived from empirical data. The first treatment threshold can be a treatment threshold for electrical stimulation pulses provided by a specific plurality of electrodes - for example electrodes **172** - directed to a specific area or areas of the scalp. In some embodiments, the first treatment threshold relates to anteriorly-placed electrodes **172** which generate electrical stimulation pulses directed to anterior region(s) of the scalp, including for example portions of the forehead. The treatment threshold can comprise a value or range of values for current intensity that are deemed to provide effective transdermal neurostimulation treatment. For example, the treatment threshold can be found above a minimum value for user-perceived paresthesia and below a maximum value for user pain and/or discomfort. In some embodiments, the values for treatment threshold are determined empirically by testing users for said minimum and maximum values.

### EXAMPLE 1

In an example, a direct relationship between a first treatment threshold and a sensory threshold was empirically established using a sample group of 24 users of a headset **100** for transdermal neurostimulation. The following table summarizes sensory thresholds *(Tto)* in milli-amperes (mA), defined in accordance with the teachings of Step **S07** for each of the users; first treatment thresholds *(Tt)* in mA; and the ratio of *Tt* to *Tto* for each user.

| User # | Gender (M/F) | *Tto* (mA) | *Tt* (mA) | *Tt*/*Tto* |
|---|---|---|---|---|
| 1 | F | 1.2 | 2.1 | 1.7 |
| 2 | F | 1.3 | 2.2 | 1.7 |
| 3 | F | 1.2 | 1.9 | 1.6 |
| 4 | F | 1.2 | 3.3 | 2.8 |
| 5 | F | 1.1 | 1.8 | 1.6 |
| 6 | F | 1.2 | 1.8 | 1.5 |
| 7 | F | 1.6 | 2.8 | 1.8 |
| 8 | F | 1.6 | 2.6 | 1.6 |
| 9 | F | 1.8 | 2.4 | 1.3 |
| 10 | F | 1.3 | 1.7 | 1.3 |
| 11 | F | 1.3 | 1.9 | 1.4 |
| 12 | F | 1.8 | 3.0 | 1.7 |
| 13 | M | 1.7 | 3.3 | 1.9 |
| 14 | M | 1.7 | 2.5 | 1.4 |
| 15 | M | 1.7 | 2.7 | 1.6 |
| 16 | M | 1.2 | 2.4 | 1.9 |
| 17 | M | 1.3 | 2.3 | 1.8 |
| 18 | M | 1.3 | 2.7 | 2.1 |
| 19 | M | 1.4 | 2.0 | 1.5 |
| 20 | M | 1.2 | 2.7 | 2.3 |
| 21 | M | 1.3 | 2.5 | 1.9 |
| 22 | M | 2.0 | 2.9 | 1.5 |
| 23 | M | 1.6 | 3.1 | 1.9 |
| 24 | M | 2.1 | 3.2 | 1.5 |

From the results for the 24 users, it can be seen that the ratio of *Tt* to *Tto* has a minimum value of 1.3, a maximum value of 2.8, and a mean of 1.7.

The first treatment threshold of Step **S08** can be calculated by multiplying the defined sensory threshold for a user by a multiplier A based on the relationship between *Tt* and *Tto* as seen in the empirical data. The multiplying factor *A,* based on the empirical data, can be set anywhere within a range of values encompassing all or most of the users' respective preferred treatment thresholds. For example, A can be in the range of 1.0 to 3.2, inclusive; a preferred treatment threshold for all 24 users in Example 1 is found within this range. Alternatively, A can be set anywhere in the range of 1.3 to 2.8, inclusive, and once again a preferred treatment threshold for all 24 users in Example 1 is found within this range. Alternatively, A can be set anywhere in the range 1.4 to 2.0, inclusive; 19 out of 24 users have optimal treatment thresholds in this range. Yet alternatively, A can be set anywhere in the range 1.3 to 1.9; 21 out of 24 users have optimal treatment threshold in this range. Alternatively or additionally, the relationship between *Tt* and *Tto* can include other electrical parameters such as any of the electrical parameters discussed hereinabove.

Steps **S07** and **S08** can be included in program instructions **51** carried out by one or more processors **54** of a user input device such as a mobile phone **570.**

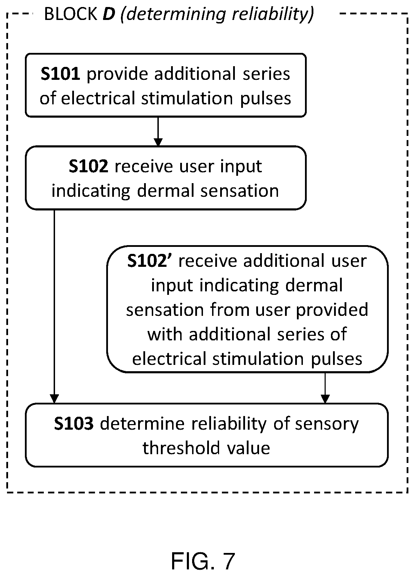

In some embodiments, it can be desirable to test the reliability of the sensory threshold, i.e., the current intensity at which the user's indication (i.e., the first user input received in Step **S06** or **S06')** of feeling the dermal sensation was received. Block ***D** (determining reliability),* illustrated in Fig. 7, comprises method steps directed to testing the reliability of the sensory threshold.

In Step **S101,** an additional series of electrical stimulation pulses is provided by a plurality of electrodes, e.g., the same plurality of electrodes used for the pulses in Step **S05.** Step **S102** comprises receiving an additional user input indicating that the user has experienced a dermal sensation, e.g., paresthesia, related to the additional series of pulses of Step **S101.**

Alternative Step **S102'** comprises receiving an additional user input indicating that the user has experienced, during an additional series of electrical stimulation pulses of increasing current intensity applied by a plurality of stimulating electrodes **102** to specific area or areas (e.g., anterior region) of the user's scalp, a dermal sensation related to the series of pulses. Step **S102'** can be included in program instructions **51** carried out by one or more processors **54** of a user input device such as a mobile phone **570.** The pulses of Step **S101** or Step **S102'** increase monotonically in current intensity as the series progresses, at a rate of increase that can be same as the rate of increase of the first series of pulses of Step **S05** or of Step **S06',** or different than that rate of increase. The user input of Step **S101** or of Step **S102'** is preferably received via a user interface **52** of mobile device **570** and/or on-board interface **180.**

Once the additional user input has been received in Step **S101** or Step **S102',** reliability of the sensory threshold value can be determined in Step **S103.** This can include comparing the current intensity at which the additional user input is received to the sensory threshold defined in Step **S07.** Steps **S103** and **S08** can be included in program instructions **51** carried out by one or more processors **54** of a user input device such as a mobile phone **570.** In some embodiments, if the additional-user-input current intensity is within ±10% of the sensory threshold defined in Step **S07,** the sensory threshold value can be determined to be reliable. In some embodiments, if the additional-user-input current intensity is within ±20% of the sensory threshold defined in Step **S07,** the sensory threshold value can be determined to be reliable. In some embodiments, if the additional-user-input current intensity is within ±30% of the sensory threshold defined in Step **S07,** the sensory threshold value can be determined to be reliable.

As will be discussed later, the method steps of Block ***D*** can be repeated any number of times in order to test the reliability of the sensory threshold for the user. If the sensory threshold value cannot be determined to be reliable after a desired number of repetitions, a treatment protocol based on default values can be drawn up and/or implemented. Default values can be modified based on stored user information.

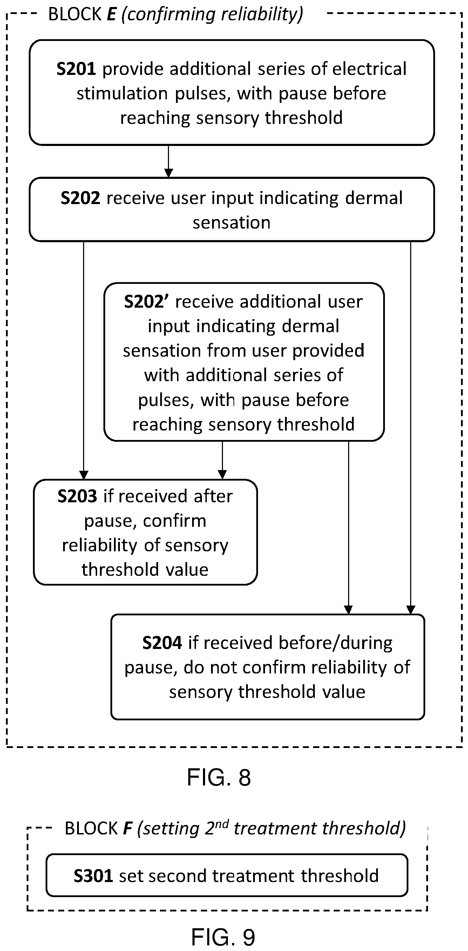

In some embodiments, it can be desirable to confirm the reliability of the sensory threshold, i.e., the current intensity at which the user's indication (i.e., the first user input received in Steps **S06** or **S06')** of feeling the dermal sensation was received. Block ***E** (confirming reliability),* illustrated in Fig. 8, comprises method steps directed to confirming the reliability of the sensory threshold.

In Step **S201,** an additional series of electrical stimulation pulses is provided by a plurality of electrodes, e.g., the same plurality of electrodes used for the pulses in Step **S05.** In some embodiments, the pulses can be provided by a different plurality of electrodes than that used for the pulses in Step **S05.** In an example, the pulses in Step **S05** can be provided by an anteriorly-placed plurality of electrodes, and the pulses in Step **S201** can be provided by a posteriorly-placed plurality of electrodes. As discussed hereinbelow, there can be a direct, and in some embodiments empirically-derived, relationship between respective set of treatment parameters set for anteriorly-placed plurality of electrodes and posteriorly-placed plurality of electrodes to complement the use of a different plurality of electrodes in Step **S201.** Step **S202** comprises receiving an additional user input indicating that the user has experienced a dermal sensation, e.g., paresthesia, related to the additional series of pulses of Step **S201.** Alternative Step **S202'** comprises receiving an additional user input indicating that the user has experienced, during an additional series of electrical stimulation pulses of increasing current intensity applied by a plurality of stimulating electrodes **102** to specific area or areas (e.g., anterior region) of the user's scalp, a dermal sensation related to the series of pulses. Step **S202'** can be included in program instructions **51** carried out by one or more processors **54** of a user input device such as a mobile phone **570.** The user input of Step **S201** or Step **S202'** is preferably received via a user interface 52 of mobile device **570** and/or on-board interface **180.**

The pulses of Step **S201** or Step **S202'** increase monotonically in current intensity as the series progresses, at a rate of increase that can be same as the rate of increase of the first series of pulses of Steps **S05 or S06',** or different than that rate of increase. In contrast to those other steps, the pulses of Step **S201** or Step **S202'** are interrupted by a pause before the current intensity increases to the level of the sensory threshold defined in Step **S07.** Preferably the pause occurs long enough before reaching the level of the sensory threshold so that the current intensity is not substantially the same as that of the sensory threshold (e.g., within 10% of the sensory threshold, within 20% of the sensory threshold, or within 30% of the sensory threshold). After the pause, which can be for less than a second, or for a second or more, or for several seconds, the series of pulses (and the increase in current intensity) continue until a user input is received in Step **S202** or in Step **S202'** indicating that the user has experienced the dermal sensation (e.g., paresthesia). If the user input of Steps **S202 or S202'** is received after the resumption of pulses following the pause (and within an acceptable range of current intensity values from the sensory threshold), then the sensory threshold is confirmed in Step **S203.** On the other hand, if the user input of Steps **S202 or S202'** is received before or during the pause, i.e., well before the pulses reached the sensory threshold defined in Step **S07,** then in Step **S204** the sensory threshold is not confirmed.

Steps **S203** and **S204** can be included in program instructions **51** carried out by one or more processors **54** of a user input device such as a mobile phone **570.**

As will be discussed later, the method steps of Block ***E*** can be performed after the steps of Block ***B*** (defining the sensory threshold) or after one or more Block ***D*** cycles/repetitions (determining the reliability of the sensory threshold). In some embodiments, it can be preferable to perform Block ***E*** before Block C (setting the first treatment threshold).

Referring now to Fig. 9, Block ***F** (setting 2^{nd} treatment threshold)* comprises Step **S301,** which includes setting a second treatment threshold based on a relationship between the second treatment threshold and the first treatment threshold. The relationship, in preferred embodiments, is a direct relationship derived from empirical data. The second treatment threshold can be a treatment threshold for electrical stimulation pulses provided by a specific plurality of electrodes - for example electrodes **174** - directed to a specific area or areas of the scalp. In some embodiments, the second treatment threshold relates to posteriorly-placed electrodes **174** which generate electrical stimulation pulses directed to the posterior region(s) of the scalp. The treatment threshold can comprise a value or range of values for current intensity that are deemed to provide effective transdermal neurostimulation treatment. For example, the treatment threshold can be found above a minimum value for user-perceived paresthesia and below a maximum value for user pain and/or discomfort. In some embodiments, the values for treatment threshold are determined empirically by testing users for said minimum and maximum values.

### EXAMPLE 2

Example 2 is an extension of Example 1 in that a direct relationship between the second treatment threshold and the first treatment threshold was empirically established using the same sample group of 24 users of a headset **100** for transdermal neurostimulation. The following table summarizes first treatment thresholds *(Tt)* in milli-amperes (mA), as defined in accordance with the teachings of Step **S07** for each of the users; second treatment thresholds *(Ot)* in mA; and the ratio of *Ot* to *Tt.*

| User # | Gender (M/F) | *Tt* (mA) | *Ot* (mA) | *Ot*/*Tt* |
|---|---|---|---|---|
| 1 | F | 2.1 | 5.0 | 2.4 |
| 2 | F | 2.2 | 4.7 | 2.1 |
| 3 | F | 1.9 | 4.4 | 2.3 |
| 4 | F | 3.3 | 6.6 | 2.0 |
| 5 | F | 1.8 | 4.2 | 2.3 |
| 6 | F | 1.8 | 4.4 | 2.4 |
| 7 | F | 2.8 | 5.7 | 2.0 |
| 8 | F | 2.6 | 5.3 | 2.0 |
| 9 | F | 2.4 | 5.5 | 2.3 |
| 10 | F | 1.7 | 5.5 | 3.2 |
| 11 | F | 1.9 | 4.7 | 2.5 |
| 12 | F | 3.0 | 5.8 | 1.9 |
| 13 | M | 3.3 | 7.8 | 2.4 |
| 14 | M | 2.5 | 6.4 | 2.6 |
| 15 | M | 2.7 | 6.5 | 2.4 |
| 16 | M | 2.4 | 5.4 | 2.3 |
| 17 | M | 2.3 | 5.4 | 2.3 |
| 18 | M | 2.7 | 5.3 | 2.0 |
| 19 | M | 2.0 | 6.7 | 3.4 |
| 20 | M | 2.7 | 5.9 | 2.2 |
| 21 | M | 2.5 | 8.0 | 3.2 |
| 22 | M | 2.9 | 7.1 | 2.4 |
| 23 | M | 3.1 | 6.9 | 2.2 |
| 24 | M | 3.2 | 6.7 | 2.1 |

From the results for the 24 users, it can be seen that the ratio of *Ot* to *Tt* has a minimum value of 1.9, a maximum value of 3.4, and a mean of 2.4.

The second treatment threshold of Step **S301** can be calculated by multiplying the first treatment threshold for a user by a multiplier *B* based on the relationship between *Ot* and *Tt* as seen in the empirical data. The multiplying factor *B,* based on the empirical data, can be set anywhere within a range of values encompassing all or most of the users' respective preferred treatment thresholds. For example, *B* can be in the range of 1.1 to 3.8, inclusive; a preferred treatment threshold for all 24 users in Example 2 is found within this range. Alternatively, *B* can be set anywhere in the range of 1.4 to 3.4, inclusive, and once again a preferred treatment threshold for all 24 users in Example 2 is found within this range. Alternatively, *B* can be set anywhere in the range 1.8 to 3.0, inclusive; 21 out of 24 users have optimal treatment thresholds in this range. Alternatively or additionally, the relationship between *Ot* and *Tt* can include other electrical parameters such as any of the electrical parameters discussed hereinabove.

Step **S301** can be included in program instructions **51** carried out by one or more processors **54** of a user input device such as a mobile phone **570.**

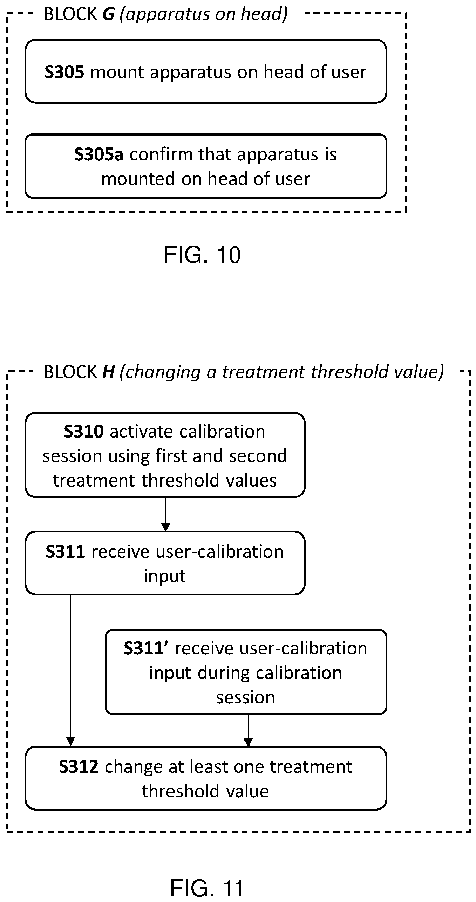

Reference is now made to Figs. 10-13, where flowcharts of method steps and 'building-blocks' of method steps relating, inter alia, to calibration of treatment thresholds are shown in accordance with various embodiments.

Referring to Fig. 10, Block ***G** (apparatus on head)* comprises Step **S305,** which includes mounting, on the head of a user, an apparatus comprising electrodes configured to provide electrical stimulation pulses, of the type used in transdermal neurostimulation treatments. In embodiments, the apparatus can be a headset such as, for example, any of the headsets **100** discussed previously in this disclosure. The apparatus can be any of the apparatuses described with respect to Fig. 4 and the discussion of Block *A* earlier in the disclosure. The apparatus can be the same apparatus used in Block *A*, and may still be in place since the performance of Block A steps (and/or any other method steps). In some embodiments, Block ***G*** additionally or alternatively comprises Step **S305a,** which includes confirming that such an apparatus is mounted on the head of a user. In some embodiments, the confirming is included in program instructions **51** carried out by one or more processors **54** of a user input device such as a mobile phone **570.** In some such embodiments, the confirming can include receiving confirming information from a sensor such as, for example sensing electrodes **104.** In other such embodiments, the confirming can include receiving a user input on a user interface **52** of mobile device **570.** The confirming can additionally include soliciting a user input on user interface **52** prior to receiving the user input. Additionally, or alternatively, the confirming can include receiving a user input via on-board interface **180.**

In some embodiments, it can be desirable to have a user calibrate the treatment thresholds set in Steps **S08** and **S301** in a dedicated calibration session. The calibration by the user can include changing a treatment protocol parameter - for example the current intensity - in order to increase the comfort level of undergoing a treatment. In some cases, a user might prefer to increase the current intensity, and in other cases she might prefer to decrease the current intensity or modify another electrical parameter. For example, decreasing the electrical charge over time migh include increasing the current intensity by 5% but shortening hte duration of the pulses by 10%. In other examples a user might wish to make the electrical stimulation pulses of the transdermal neurostimulation treatment shorter or longer, and/or more frequent or infrequent. In some embodiments, the system **101** can be configured to receive such calibration-inputs from a user and, in response, make the requested changes very quickly (e.g., within less than a second after the user finishes making changes, or less than 500 milliseconds, or less than 200 milliseconds). In some embodiments, the system **101** can be configured to adjust - in response to the user-calibration inputs - not only the value of the parameter directly changed by the user, but also the value of at least one additional parameter. For example, if a user changes the value of a current intensity treatment protocol parameter, the system **101** may be configured to adjust, for example, one or more of pulse width (duration), phase width, and/or frequency. The at one or more additional parameter may be automatically changed in response to the user-calibration input, in order to serve a treatment goal. For example, it can be a treatment goal to maintain an overall average current such that the overall average current during a treatment stays at a predetermined value. (One way of calculating average current is to multiply current intensity by the ratio of phase width to pulse width, with average current typically being measured in milliamperes.) Thus, decreasing current intensity could trigger an increase in phase width. As another example, it can be a treatment goal to maintain average current within an acceptable variance (e.g., ±30%, ±20% or ±10%), of a predetermined value. In both of these examples, the predetermined value can be the overall average current value before the user-calibration inputs.

In some embodiments, a dedicated user calibration session can take place immediately before a treatment session, and in other embodiments the calibration session can be a standalone interaction with the system **101** and its components such as headset **100,** with mobile device **570** serving as user-input device by executing, on command program instructions **51** by the one or more processors **54.** In some embodiments, a calibration session can take place immediately or soon after the performance of Step **S301** (setting the second treatment threshold).

Referring now to Fig. 11, Block ***H** (changing a treatment threshold value)* comprises a group of method steps related to user calibration of treatment thresholds.

In Step **S310,** a calibration session is activated; in some embodiments the base (initial) treatment threshold values are those set in Steps **S08** and **S301** and in some other embodiments one or both of the initial treatment threshold values has already been changed in a previous calibration session. A calibration can be initiated by a user, or it can be initiated by the system running an app (executing program instructions **51)** on a user input device (e.g., mobile device **570).** According to embodiments, electrical stimulation pulses are delivered to two portions of the user's scalp (e.g., the anterior and posterior portions) during the calibration session.

Step **S311** comprises receiving a user-calibration input from the user, where a user-initiated change can be made to the value of any treatment parameter as discussed above. Alternative Step **S311'** comprises receiving a user-calibration input after the activation of a calibration session using first and second current-intensity treatment thresholds and/or other electrical parameter thresholds as respective initial current intensities applied to two portions of the user's scalp (e.g., the anterior and posterior portions), or, equivalently, using current-intensity treatment thresholds that have been previously changed by the user in an earlier calibration session. All user inputs, sensory thresholds, treatment thresholds, user-calibration inputs and other data related to and/or generated by the system **101** can be stored in any one of the external devices **150** as well as on external servers in the cloud (not shown). Step **S311'** can be included in program instructions **51** carried out by one or more processors **54** of a user input device such as a mobile phone **570.** The user-calibration inputs of Step **S311** or Step **S311'** are preferably received via a user interface **52** of mobile device **570** and/or on-board interface **180.**

In response to receiving a user-calibration input during a treatment session (Step **S311** or **S311'),** a change is effected, in Step **S312,** to at least one treatment threshold value. As discussed earlier, one or more other parameters may be modified so as to maintain treatment efficacy, for example by keeping overall average current within a predetermined range. Step **S312** can be included in program instructions **51** carried out by one or more processors **54** of a user input device such as a mobile phone **570.**

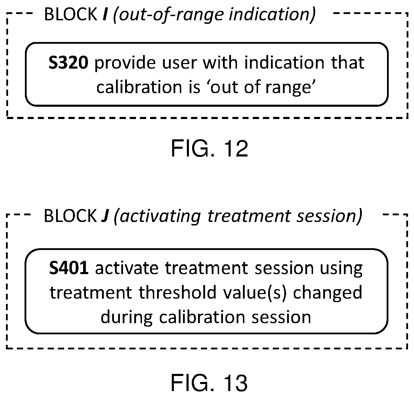

We now refer to Fig. 12, which shows method-step Block ***I** (out-of-range indication)* comprising Step **S320.** In some embodiments, Step **S320** alternatively or additionally includes, in response to receiving a user-calibration input in Step **S311** or **S311',** providing the user with an indication that the change request embodied in a calibration-user input received during the calibration session will take the treatment parameters outside of the recommended range for effective neurostimulation treatment. A user receiving such an indication, e.g. through the user interface **52** of mobile device **570** can be given an opportunity to 'correct' the calibration-user input so as to remain within the recommend range of parameter values. Step **S320** can be included in program instructions **51** carried out by one or more processors **54** of a user input device such as a mobile phone **570.**

Block ***J** (activating treatment session),* illustrated in Fig. 13, comprises Step **S401,** which involves activating a transdermal neurostimulation treatment session using one or more treatment parameters changed in a calibration session.

Reference is made to Figs. 14-17, which illustrate examples of methods, according to embodiments, that include method steps building blocks discussed with reference to Figs. 4-13.

Figs. 14A-D show several options for setting treatment thresholds in accordance with the various embodiments.

In Fig. 14A, an exemplary method is shown to include mounting a headset / confirming that the headset is mounted (Block *A*), defining a sensory threshold (Block ***B*),** setting a first treatment threshold (Block *C*) and setting a second treatment threshold (Block ***F*).**

In Fig. 14B, another exemplary method is shown to include mounting a headset / confirming that the headset is mounted (Block ***A***), defining a sensory \threshold (Block ***B**),* determining the reliability of the sensory threshold (Block ***D**),* setting a first treatment threshold (Block ***C***) and setting a second treatment threshold (Block ***F***).

In Fig. 14C, yet another exemplary method is shown to include mounting a headset / confirming that the headset is mounted (Block ***A***), defining a sensory threshold (Block ***B***), confirming the reliability of the sensory threshold (Block ***E***), setting a first treatment threshold (Block ***C***) and setting a second treatment threshold (Block ***F***).

In Fig. 14D, yet another exemplary method is shown to include mounting a headset / confirming that the headset is mounted (Block ***A***), defining a sensory threshold (Block ***B**),* determining the reliability of the sensory threshold (Block ***D**),* confirming the reliability of the sensory threshold (Block ***E***), setting a first treatment threshold (Block ***C***) and setting a second treatment threshold (Block ***F***).

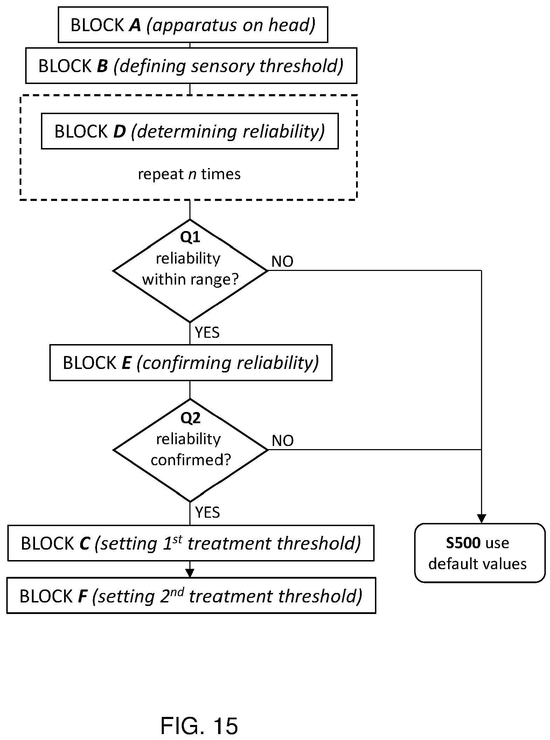

Fig. 15 illustrates another exemplary method, that includes mounting a headset / confirming that the headset is mounted (Block ***A***); defining a sensory threshold (Block ***B**);* and determining the reliability of the sensory threshold (Block ***D**),* with the Block ***D*** method steps repeated *n* times, with *n* being a integer greater than 1 *(n* is not greater than 10 in some embodiments, not greater than 8 in other embodiments, and not greater than 6 in still other embodiments). If after *n* repetitions, the reliability is not determined in Step **S103** to be within a predetermined range, e.g., the sensory threshold is repeated with accuracy of +30%, ±20% or ±10% (branch **Q1**), then Step **S500** is carried out, which involves using a set of system default values as initial (pre-calibration) treatment threshold values. If **Q1** is resolved positively, then the method of Fig. 15 continues with confirming the reliability of the sensory threshold (Block ***E*).** If the reliability cannot be confirmed (i.e., Step **S204** has been carried out) then Step **S500** (reverting to default initial treatment threshold values) is performed. If Q2 is resolved positively, then performing the method additionally includes carrying out the method steps of setting a first treatment threshold (Block ***C***) and setting a second treatment threshold (Block ***F***).

In some embodiments, other combinations of the disclosed method steps and method-step building blocks may be performed, and all of them are within the scope of the present disclosure. It is mentioned for the sake of clarity that the method steps of Block ***A*** can be carried out whenever there is a discontinuity in the performance of any of the exemplary methods.

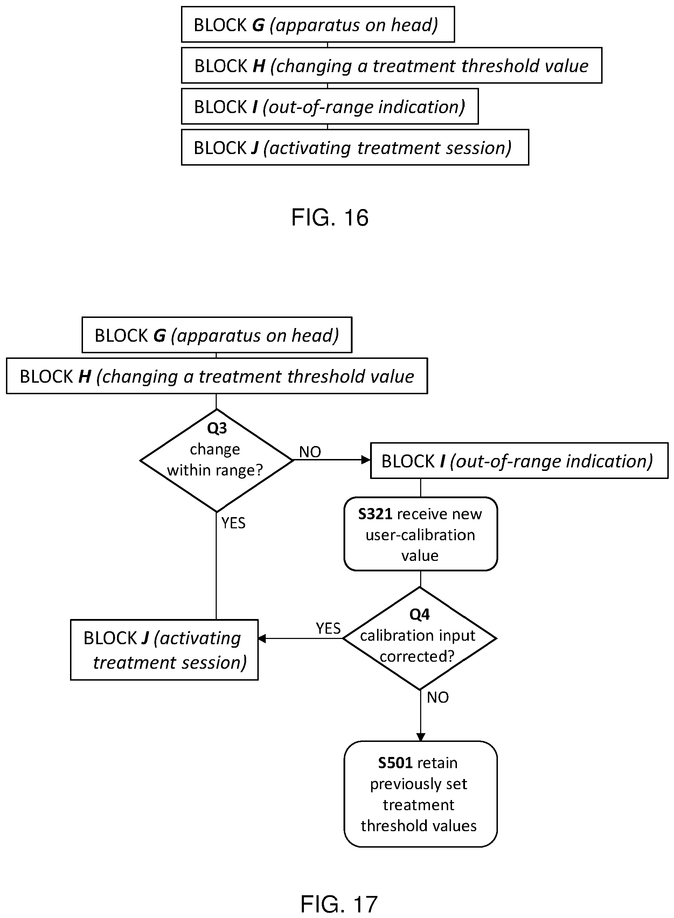

Figs. 16-17 illustrate options for completing calibration of treatment thresholds in accordance with the various embodiments.

In Fig. 16, an exemplary method is shown to include mounting a headset / confirming that the headset is mounted (Block ***G***), changing at least one treatment threshold value in a calibration session (Block ***H***), indicating that a user-calibration input would take values out of range (Block ***I***), and activating treatment session based on one or more values changed in a calibration session (Block ***J***). Not all steps and blocks of the method need be performed in all embodiments. For example, in some embodiments Block ***G*** is performed only if the method is not performed consecutively following one of the methods of Figs. 13-15 (or equivalent) in which the headset is already in place. Block ***I*** is performed only if a received user-calibration input would take the treatment parameters out of the desired range. Block ***J*** is optional, as a calibration session can be performed without continuing directly into an actual transdermal neurostimulation session.

Fig. 17 illustrates another exemplary method, that optionally includes mounting a headset / confirming that the headset is mounted (Block ***G***), and includes changing at least one treatment threshold value in a calibration session (Block ***H***)*.* If (in **Q3)** a received input for a change is out of range, then Block ***I*** is carried out. If (in **Q3)** no received input for a change is out of range, then the method continues and Block ***J*** (activating an actual treatment session with at least one changed treatment parameter) can optionally be carried out. If Block **I** is invoked in branching at **Q3,** then Step **S321,** receiving a new user-calibration value, can be performed. If (at **Q4)** a new or 'correct' (in range) user-calibration input has been received in Step **S321,** then Block ***J*** (activating an actual treatment session with at least one changed treatment parameter) can optionally be carried out; otherwise (i.e., if it is determined at **Q4)** that a new/correct/in-range user-calibration has not been received, then the system defaults, in Step S501 to retaining previously-set treatment threshold values.

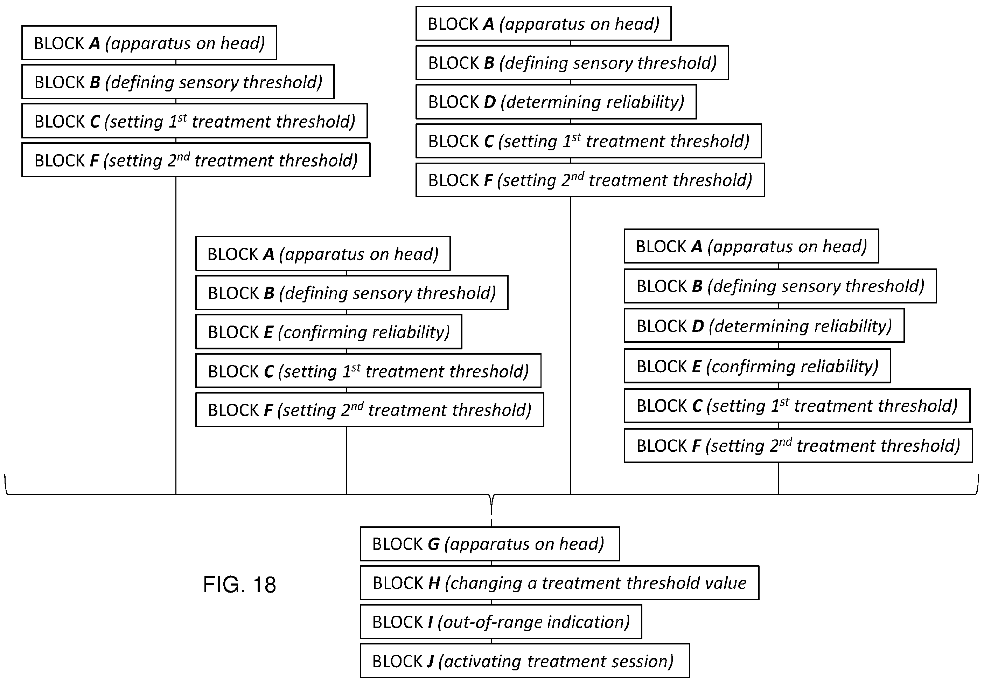

Figure 18 illustrates, schematically, another exemplary method, in which any one of the four methods described with reference to Figs. 14A-D, respectively, can be combined 'end-to-end' with the method of Fig. 16.

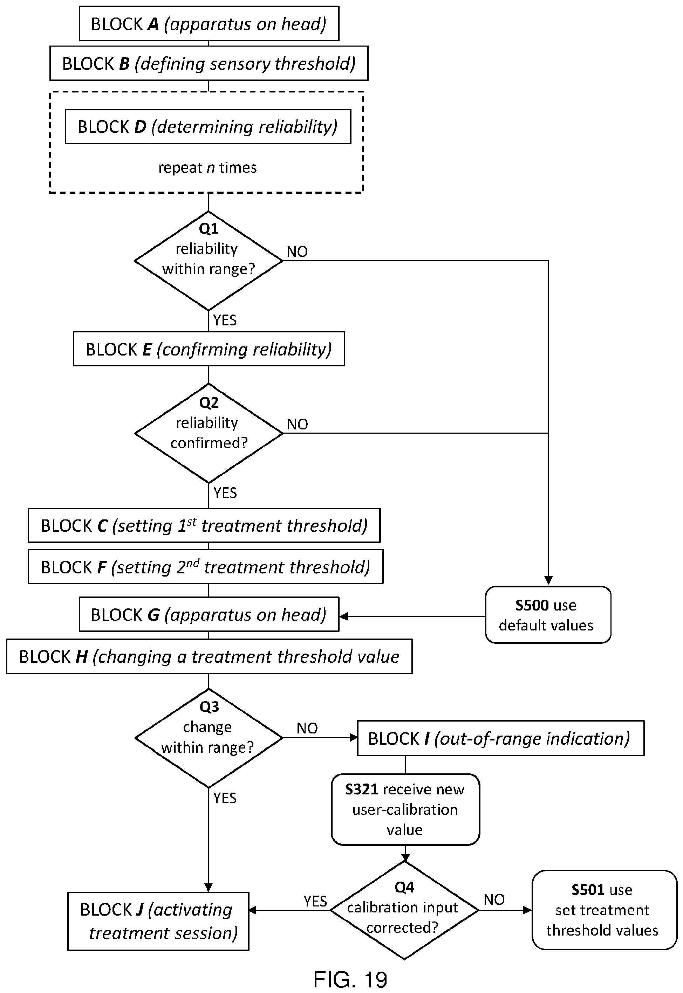

Figure 19 illustrates, also schematically, another exemplary method, in which the method of Fig. 15 can be combined 'end-to-end' with the method of Fig. 17.

Any of the methods illustrated in Figs. 13-19, or their equivalent, can be included in program instructions **51** (e.g., for an app) for execution by one or more processors **54** of a mobile device **570.**

It will be obvious to the skilled artisan that the examples and embodiments described herein are not limited to a 'first set the anterior threshold, then set the posterior threshold' paradigm as has been employed for the sake of convenience throughout much of this disclosure.

As an example, a sensory threshold may be determined using a posteriorly-directed set of electrodes, that sensory threshold then 'translated' to a posterior treatment threshold, and that posterior treatment threshold 'translated' to an anterior treatment threshold, both 'translations' being based on an empirically-derived relationship between the respective current intensity thresholds.

As another example, any number of different cranial or peripheral nerves targeted for transdermal neurostimulation treatment can be addressed in a desired order, and treatment thresholds may be set in accordance with empirically-derived relationships between the various regions or nerves (or nerve groups) in that desired order. In some embodiments, the targeted nerve branches can be laterally located on the left and right sides of the user's head.

Following are examples of the order in which treatment thresholds can be determined for different nerve branches or regions of the user's scalp, for two pluralities of electrodes targeting two regions of the scalp (A=anterior; P=posterior; LL=left lateral; RL=right lateral): : A-P; A-LL; A-RL; P-A; P-LL; -RL; LL-A; LL-P; LL-RL; RL-A; RL-P; and RL-LL. Obviously when three or more regions of the scalp are targeted and corresponding numbers of electrodes are used, the number of possible combinations increases accordingly.

It should also be clear that the embodiments are not limited to a user's head, and may be applied to any limb or region of the body for which empirical relationships can be derived between sensory thresholds and treatment thresholds, and further between different treatment thresholds.

Any such variations applied to the methods and devices disclosed herein are clearly within the scope of the present disclosure.

As used herein, wherever it is written that a given method step or a given group of method steps can be 'included' in program instructions, it should be understood that instructions to perform the given method step or given group of method steps can be included in said program instructions, such that execution of said program instructions (e.g., by one or more computer processors or one or more processors of a mobile device, as appropriate) causes the one or more processors to perform the given method step or given group of method steps, or causes the one or more processors to cause the given method step or given group of method steps to be carried out, as appropriate.

As used herein in the specification and in the claims section that follows, the term "or" is considered as inclusive, and therefore the phrase "A or B" means any of the groups "A", "B", and "A and B".

As used herein in the specification and in the claims section that follows, the term "pulse" relates to an electrical signal, for example applied via an electrode or sensed by an electrode.

It will be appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub-combination. Similarly, the content of a claim depending from one or more particular claims may generally depend from the other, unspecified claims, or be combined with the content thereof, absent any specific, manifest incompatibility therebetween.

## Claims

1. A non-therapeutic method of determining user-specific treatment parameter values for a multi-channel transdermal neurostimulation treatment applied at multiple locations on a user's scalp, the method comprising:
a. setting a first set of treatment parameter values for a first electrode array in engagement with a first portion of the user's scalp for administering electrical pulses thereto; and
b. setting a second set of treatment parameter values for a second electrode-array in engagement with a second portion of the user's scalp for administering electrical pulses thereto, according to a second correlation that includes a direct relationship between at least one parameter value of said second set of treatment parameter values and said at least one parameter value of said first set of treatment parameter values,
**characterised in that** either (i) said first portion of the scalp is an anterior portion and said second portion of the scalp is a posterior portion, or (ii) said first portion of the scalp is a posterior portion and said second portion of the scalp is an anterior portion.

2. The method of claim 1, wherein each respective set of treatment parameter values includes values for at least two of:
i. an electrical charge parameter having values of total electric charge for a given time period, where the given time period is an integer multiple of an inverse of electrical pulse frequency,
ii. a current-intensity parameter, and
iii. a pulse duration parameter and a frequency parameter, or an arithmetic combination thereof.

3. The method of any one of claims 1 to 2, wherein said direct relationship is based in part on a relationship between respective electrode surface-areas of said first and second electrode arrays.

4. The method of any one of claims 1 to 3, wherein said second correlation is a linear relationship based on an empirical correlation.

5. The method of any one of claims 1 to 4, wherein according to said direct relationship, an electrical charge parameter value of said second set of treatment parameter values is equal to an electrical charge parameter value of said first set of treatment parameter values multiplied by B, where B is not less than 1.1 and not greater than 3.8.

6. The method of any one of claims 1 to 5, wherein the transdermal neurostimulation treatment includes anteriorly-applied electrical pulses targeting the trigeminal nerve and posteriorly-applied electrical pulses targeting the occipital nerve.

7. The method of any one of claims 1 to 6, wherein the setting of the first set of treatment parameter values is according to a first correlation that includes a first direct relationship between at least one parameter value of said first set of treatment parameter values and a corresponding at least one parameter value of a set of use-specific sensory-threshold parameter values.

8. The method of claim 7, wherein values of the set of user-specific sensory-threshold parameter values are based on a first user input received during application of a series of electrical pulses to a single one of the first and second portions of the user's scalp.

9. The method of claim 7 or claim 8, wherein said first correlation is a linear relationship based on an empirical correlation.

10. The method of any one of claims 7 to 9, wherein according to said first direct relationship, an electrical charge parameter value of said first set of treatment parameter values is equal to an electrical charge parameter value of said set of user-specific sensory-threshold parameter values multiplied by A, where A is not less than 1.3 and not greater than 1.9.

11. The method of any of the preceding claims 7-10, wherein said setting of said first set of treatment parameter values comprises providing, to the user's scalp, a first series of electrical pulses having electrical parameter values that increase at a first rate of increase, wherein said respective electrical parameter values comprise at least one of an electrical charge parameter value and a current-intensity value;
b. responsively to receiving a first user input indicating that the user has felt a dermal sensation related to the application of said first series of electrical pulses, defining, as a set of sensory-threshold parameter values, a plurality of respective electrical parameter values at which said first user input is received;
c. providing, to the user's scalp, a reliability-confirming series of electrical pulses having respective electrical parameter values that increase at a second rate of increase, wherein said respective electrical parameter values comprise at least one of an electrical charge parameter value and a current-intensity value, the providing being such that said reliability-confirming series is (i) paused before respective electrical parameter values reach said sensory -threshold parameter values, and (ii) subsequently resumed after a pause;
d. responsively to receiving, after said pause and at respective electrical parameter values within a predetermined range of values surrounding said sensory -threshold parameter values, a second user input indicating that the user has felt a dermal sensation related to the application of said reliability-confirming series of electrical pulses: confirming the reliability of said sensory -threshold parameter values; and
e. applying the multi-channel transdermal neurostimulation treatment using treatment parameter values based on said reliability-confirmed sensory -threshold parameter values.

## Patentansprüche

1. Nicht-therapeutisches Verfahren zum Bestimmen benutzerspezifischer Behandlungsparameterwerte für eine mehrkanalige transdermale Neurostimulationsbehandlung, die an mehreren Stellen auf der Kopfhaut eines Benutzers angewendet wird, wobei das Verfahren umfasst:
a. Einstellen eines ersten Satzes von Behandlungsparameterwerten für eine erste Elektrodenanordnung in Kontakt mit einem ersten Abschnitt der Kopfhaut des Benutzers, um diesem elektrische Impulse zu verabreichen; und
b. Einstellen eines zweiten Satzes von Behandlungsparameterwerten für eine zweite Elektrodenanordnung in Kontakt mit einem zweiten Abschnitt der Kopfhaut des Benutzers, um diesem elektrische Impulse zu verabreichen, gemäß einer zweiten Korrelation, die eine direkte Beziehung zwischen mindestens einem Parameterwert des zweiten Satzes von Behandlungsparameterwerten und dem mindestens einen Parameterwert des ersten Satzes von Behandlungsparameterwerten beinhaltet,
**dadurch gekennzeichnet, dass** entweder (i) der erste Abschnitt der Kopfhaut ein vorderer Abschnitt ist und der zweite Abschnitt der Kopfhaut ein hinterer Abschnitt ist, oder (ii) der erste Abschnitt der Kopfhaut ein hinterer Abschnitt ist und der zweite Abschnitt der Kopfhaut ein vorderer Abschnitt ist.

2. Verfahren nach Anspruch 1, wobei jeder jeweilige Satz von Behandlungsparameterwerten Werte für mindestens zwei der folgenden beinhaltet:
i. einen elektrischen Ladungsparameter mit Werten der gesamten elektrischen Ladung für eine gegebene Zeitspanne, wobei die gegebene Zeitspanne ein ganzzahliges Vielfaches eines Kehrwerts der elektrischen Impulsfrequenz ist,
ii. einen Stromintensitätsparameter und
iii. einen Impulsdauerparameter und einen Frequenzparameter oder eine arithmetische Kombination davon.

3. Verfahren nach einem der Ansprüche 1 bis 2, wobei die direkte Beziehung teilweise auf einer Beziehung zwischen jeweiligen Elektrodenoberflächenbereichen der ersten und der zweiten Elektrodenanordnung beruht.

4. Verfahren nach einem der Ansprüche 1 bis 3, wobei die zweite Korrelation eine lineare Beziehung ist, die auf einer empirischen Korrelation beruht.

5. Verfahren nach einem der Ansprüche 1 bis 4, wobei gemäß der direkten Beziehung ein elektrischer Ladungsparameterwert des zweiten Satzes von Behandlungsparameterwerten gleich einem elektrischen Ladungsparameterwert des ersten Satzes von Behandlungsparameterwerten multipliziert mit B ist, wobei B nicht kleiner als 1,1 und nicht größer als 3,8 ist.

6. Verfahren nach einem der Ansprüche 1 bis 5, wobei die transdermale Neurostimulationsbehandlung anterior verabreichte elektrische Impulse, die auf den Trigeminusnerv abzielen, und posterior verabreichte elektrische Impulse, die auf den Okzipitalnerv abzielen, beinhaltet.

7. Verfahren nach einem der Ansprüche 1 bis 6, wobei das Einstellen des ersten Satzes von Behandlungsparameterwerten gemäß einer ersten Korrelation erfolgt, die eine erste direkte Beziehung zwischen mindestens einem Parameterwert des ersten Satzes von Behandlungsparameterwerten und einem entsprechenden mindestens einen Parameterwert eines Satzes von nutzungsspezifischen Wahrnehmungsschwellen-Parameterwerten beinhaltet.

8. Verfahren nach Anspruch 7, wobei Werte des Satzes von benutzerspezifischen Wahrnehmungsschwellen-Parameterwerten auf einer ersten Benutzereingabe beruhen, die während des Verabreichens einer Serie von elektrischen Impulsen an einen einzelnen von dem ersten und dem zweiten Abschnitt der Kopfhaut des Benutzers empfangen wird.

9. Verfahren nach Anspruch 7 oder Anspruch 8, wobei die erste Korrelation eine auf einer empirischen Korrelation beruhende lineare Beziehung ist.

10. Verfahren nach einem der Ansprüche 7 bis 9, wobei gemäß der ersten direkten Beziehung ein elektrischer Ladungsparameterwert des ersten Satzes von Behandlungsparameterwerten gleich einem elektrischen Ladungsparameterwert des Satzes von benutzerspezifischen Wahrnehmungsschwellen-Parameterwerten, multipliziert mit A, ist, wobei A nicht kleiner als 1,3 und nicht größer als 1,9 ist.

11. Verfahren nach einem der vorhergehenden Ansprüche 7 bis 10, wobei das Einstellen des ersten Satzes von Behandlungsparameterwerten das Verabreichen einer ersten Serie von elektrischen Impulsen an die Kopfhaut des Benutzers umfasst, welche elektrische Parameterwerte aufweisen, die mit einer ersten Steigerungsrate ansteigen, wobei die jeweiligen elektrischen Parameterwerte mindestens einen von einem elektrischen Ladungsparameterwert und einem Stromintensitätswert umfassen;
b. als Reaktion auf den Empfang einer ersten Benutzereingabe, die angibt, dass der Benutzer ein Hautgefühl im Zusammenhang mit der Anwendung der ersten Serie von elektrischen Impulsen gespürt hat, Definieren einer Vielzahl von jeweiligen elektrischen Parameterwerten, bei denen die erste Benutzereingabe empfangen wird, als einen Satz von Wahrnehmungsschwellen-Parameterwerten,
c. Verabreichen einer zuverlässigkeitsgeprüften Serie von elektrischen Impulsen an die Kopfhaut des Benutzers, welche jeweilige elektrische Parameterwerte aufweisen, die mit einer zweiten Steigerungsrate ansteigen, wobei die jeweiligen elektrischen Parameterwerte mindestens eines von einem elektrischen Ladungsparameterwert und einem Stromintensitätswert umfassen, wobei das Verabreichen derart erfolgt, dass die zuverlässigkeitsgeprüfte Serie (i) angehalten wird, bevor jeweilige elektrische Parameterwerte die Wahrnehmungsschwellen-Parameterwerte erreichen, und die (ii) anschließend nach einem Anhalten wieder aufgenommen wird;
d. als Reaktion auf das Empfangen, nach dem Anhalten und bei jeweiligen elektrischen Parameterwerten innerhalb eines vorbestimmten Bereichs von Werten um die Wahrnehmungsschwellen-Parameterwerte herum, einer zweiten Benutzereingabe, die angibt, dass der Benutzer ein Hautgefühl im Zusammenhang mit der Anwendung der zuverlässigkeitsgeprüften Serie von elektrischen Impulsen gespürt hat: Bestätigen der Zuverlässigkeit der Wahrnehmungsschwellen-Parameterwerte; und
e. Anwenden der mehrkanaligen transdermalen Neurostimulationsbehandlung unter Verwendung von Behandlungsparameterwerten beruhend auf den zuverlässigkeitsgeprüften Wahrnehmungsschwellen-Parameterwerten.

## Revendications

1. Procédé non thérapeutique de détermination de valeurs de paramètres de traitement spécifiques à l'utilisateur pour un traitement par neurostimulation transdermique multicanal appliqué en plusieurs endroits sur le cuir chevelu d'un utilisateur, le procédé comprenant :
a. la définition d'un premier ensemble de valeurs de paramètres de traitement pour un premier réseau d'électrodes en prise avec une première partie du cuir chevelu de l'utilisateur en vue de l'administration d'impulsions électriques à celui-ci ; et
b. la définition d'un second ensemble de valeurs de paramètres de traitement pour un second réseau d'électrodes en prise avec une seconde partie du cuir chevelu de l'utilisateur en vue de l'administration d'impulsions électriques à celui-ci, selon une seconde corrélation qui comprend une relation directe entre au moins une valeur de paramètre dudit second ensemble de valeurs de paramètres de traitement et ladite au moins une valeur de paramètre dudit premier ensemble de valeurs de paramètres de traitement,
**caractérisé en ce que** soit (i) ladite première partie du cuir chevelu est une partie antérieure et ladite seconde partie du cuir chevelu est une partie postérieure, soit (ii) ladite première partie du cuir chevelu est une partie postérieure et ladite seconde partie du cuir chevelu est une partie antérieure.

2. Procédé selon la revendication 1, chaque ensemble respectif de valeurs de paramètres de traitement comprenant des valeurs pour au moins deux paramètres parmi :
i. un paramètre de charge électrique présentant des valeurs de charge électrique totale pour une période de temps donnée, la période de temps donnée étant un multiple entier de l'inverse de la fréquence d'impulsion électrique,
ii. un paramètre d'intensité de courant, et
iii. un paramètre de durée d'impulsion et un paramètre de fréquence, ou une combinaison arithmétique de ceux-ci.

3. Procédé selon l'une quelconque des revendications 1 à 2, ladite relation directe étant basée en partie sur une relation entre les surfaces spécifiques d'électrode respectives desdits premier et second réseaux d'électrodes.

4. Procédé selon l'une quelconque des revendications 1 à 3, ladite seconde corrélation étant une relation linéaire basée sur une corrélation empirique.

5. Procédé selon l'une quelconque des revendications 1 à 4, conformément à ladite relation directe, la valeur de paramètre de charge électrique dudit second ensemble de valeurs de paramètres de traitement étant égale à une valeur de paramètre de charge électrique dudit premier ensemble de valeurs de paramètres de traitement multipliée par B, B n'étant pas inférieure à 1,1 et pas supérieure à 3,8.

6. Procédé selon l'une quelconque des revendications 1 à 5, ledit traitement par neurostimulation transdermique comprenant des impulsions électriques appliquées antérieurement ciblant le nerf trijumeau et des impulsions électriques appliquées postérieurement ciblant le nerf occipital.

7. Procédé selon l'une quelconque des revendications 1 à 6, ladite définition du premier ensemble de valeurs de paramètres de traitement étant conforme à une première corrélation qui comprend une première relation directe entre au moins une valeur de paramètre dudit premier ensemble de valeurs de paramètres de traitement et au moins une valeur de paramètre correspondante d'un ensemble de valeurs de paramètres de seuil sensoriel spécifiques à l'utilisation.

8. Procédé selon la revendication 7, les valeurs de l'ensemble de valeurs de paramètres de seuil sensoriel spécifiques à l'utilisateur étant basées sur une première entrée d'utilisateur reçue pendant l'application d'une série d'impulsions électriques sur une seule des première et seconde parties du cuir chevelu de l'utilisateur.

9. Procédé selon la revendication 7 ou la revendication 8, ladite première corrélation étant une relation linéaire basée sur une corrélation empirique.

10. Procédé selon l'une quelconque des revendications 7 à 9, conformément à ladite première relation directe, la valeur de paramètre de charge électrique dudit premier ensemble de valeurs de paramètre de traitement étant égale à la valeur de paramètre de charge électrique dudit ensemble de valeurs de paramètres de seuil sensoriel spécifique à l'utilisateur multipliée par A, A n'étant pas inférieure à 1,3 et pas supérieure à 1,9.

11. Procédé selon l'une quelconque des revendications précédentes 7 à 10, ledit réglage dudit premier ensemble de valeurs de paramètres de traitement comprenant la fourniture, au cuir chevelu de l'utilisateur, d'une première série d'impulsions électriques présentant des valeurs de paramètres électriques qui augmentent à un premier taux d'augmentation, lesdites valeurs de paramètres électriques respectives comprenant au moins une valeur parmi une valeur de paramètre de charge électrique et une valeur d'intensité de courant ;
b. en réponse à la réception d'une première entrée d'utilisateur indiquant que l'utilisateur a ressenti une sensation dermique liée à l'application de ladite première série d'impulsions électriques, la définition, en tant qu'ensemble de valeurs de paramètres de seuil sensoriel, d'une pluralité de valeurs de paramètres électriques respectives auxquelles ladite première entrée d'utilisateur est reçue ;
c. la fourniture, au cuir chevelu de l'utilisateur, d'une série d'impulsions électriques de confirmation en termes de fiabilité présentant des valeurs de paramètres électriques respectives qui augmentent à un second taux d'augmentation, lesdites valeurs de paramètres électriques respectives comprenant au moins une valeur parmi une valeur de paramètre de charge électrique et une valeur d'intensité de courant, la fourniture étant telle que ladite série de confirmation en termes de fiabilité est (i) mise en pause avant que les valeurs de paramètres électriques respectives n'atteignent lesdites valeurs de paramètres de seuil sensoriel, et (ii) reprise par la suite après une pause ;
d. en réponse à la réception, après ladite pause et à des valeurs de paramètres électriques respectives dans une plage prédéfinie de valeurs entourant lesdites valeurs de paramètres de seuil sensoriel, d'une seconde entrée d'utilisateur indiquant que l'utilisateur a ressenti une sensation dermique liée à l'application de ladite série d'impulsions électriques confirmées en termes de fiabilité : la confirmation de la fiabilité desdites valeurs de paramètres de seuil sensoriel ; et
e. l'application du traitement par neurostimulation transdermique multicanal en utilisant des valeurs de paramètres de traitement basées sur lesdites valeurs de paramètres de seuil sensoriel confirmées en termes de fiabilité.
